Europäisches Patentamt

European Patent Office

Office européen des brevets

(19)

(11) Publication number: **0 162 054 B1**

## EUROPEAN PATENT SPECIFICATION
### published in accordance with Art. 158(3) EPC

(45) Date of publication of patent specification: 06.03.91

(51) Int. Cl.⁵: **C12N 15/41**, C12Q 1/70, C12P 21/02, A61K 39/29, A61K 39/42

(21) Application number: 84903735.3

(22) Date of filing: 27.09.84

(86) International application number: PCT/US84/01552

(87) International publication number: WO 85/01517 (11.04.85 85/09)

(54) **PRODUCTION OF cDNA REPRESENTING HEPATITIS A VIRAL SEQUENCES.**

(30) Priority: 30.09.83 US 537911

(43) Date of publication of application:
27.11.85 Bulletin 85/48

(45) Publication of the grant of the patent:
06.03.91 Bulletin 91/10

(84) Designated Contracting States:
AT BE CH DE FR GB LI LU NL SE

(56) References cited:
EP-A- 44 710
EP-A- 154 587
EP-A- 0 061 740
WO-A-82/03632

Chemical Abstracts, vil. 100, no. 1, 02.01.1984
Columbus Ohio, US - K. Von der Helm et
al.:"Cloning of hepatitis A virus genome",
see page 133, abstract no. 1500h.& Biol.
Prod. Viral Dis. Munich Symp. Microbiol., 6th
1981 91-9

(73) Proprietor: **MASSACHUSETTS INSTITUTE OF TECHNOLOGY**
77 Massachusetts Avenue
Cambridge, MA 02139(US)

(72) Inventor: **TICEHURST, John, R.**
4421 Puller Drive
Kensington, MD 20895(US)
Inventor: **BALTIMORE, David**
28 Donnell Street
Cambridge, MA 02139(US)
Inventor: **FEINSTONE, Stephen, M.**
3021 Cathedral Avenue, N.W.
Washington, DC 20008(US)
Inventor: **PURCELL, Robert, H.**
17517 White Grounds Road
Boyds, MD 20841(US)

Proceedings Natl. Acad. Sci. USA, vol. 80. October 1983, Biochemistry J.R. Ticehurst et al. "Molecular cloning and characterization of hepatitis A virus cDNA", pages 5885-5889.

Inventor: **RACANIELLO, Vincent, R.**
152 East 94th Street
New York, NY 10038(US)
Inventor: **BAROUDY, Bahige, M.**
P.O. Box 4338
Chevy Chase, MD 20815(US)

(74) Representative: **Holdcroft, James Gerald, Dr.**
et al
Graham Watt & Co., Riverhead
Sevenoaks, Kent TN13 2BN(GB)

## Description

Technical Field

This invention is in the field of microbiology and more specifically relates to recombinant DNA techniques for producing genetically-engineered microorganisms.

Background Art

Hepatitis A virus (HAV) is an important cause of human hepatitis. In the United States it has been estimated that over 100,000 clinical cases occur annually. HAV continues to be endemic in underdeveloped areas of the world where infections usually occur in children, and nearly all of the young adult population have antibody to HAV (anti-HAV). Clinical hepatitis A and prevalence of anti-HAV are decreasing in industrialized nations, resulting in increasing numbers of adults susceptible to infection.

HAY is spread predominately by the fecal-oral route. Spread of hepatitis A is usually associated with overcrowding, poor hygiene, or breakdown in normal sanitary conditions. Contaminated food or water are frequent vehicles of spread. Groups at high risk include institutionalized persons, contacts of very young children in day-care centers, male homosexuals, consumers of raw shellfish and travelers to areas of the world where the disease is endemic.

The host range of HAV is limited to man, apes (especially the chimpanzee), and several species of New World monkeys. The incubation period for natural infections with HAV in man ranges from 15 to 45 days and averages 25 days. The first serological marker to appear is HAV in the stool, which often occurs 7-10 days before the onset of symptoms (dark urine or jaundice). Viral replication appears to be limited to the liver and excretion into the stool, where the highest levels of infectious virus are found, probably occurs via the biliary system. The virus is often rapidly cleared after the onset of symptoms and becomes undetectable in the stool. However, in some individuals, HAV can be found in the stool for longer periods.

Radioimmunoassay is a sensitive technique for detecting HAY in stool samples during the period of excretion, but it is not used in most clinical laboratories because assays for anti-HAV in serum (described below) are more easily performed and accurate. Therefore, patients with hepatitis A are usually considered as potentially infectious for up to two weeks after the onset of jaundice. Hepatitis A usually resolves with weeks, but occasionally illness may persist for several months. Mortality from hepatitis A or associated chronic liver disease are very unusual occurrences. Anti-HAV is almost always detectable in serum when symptoms begin. Because of this, diagnosis of hepatitis A is established using commercially available assays that are based on detection of anti-HAV IgM. An example of such an assay is that produced and marketed by Abbott Laboratories, North Chicago, IL under the name HAVAB-M[TM] kit. The development of anti-HAV IgG appears to be associated with lifelong immunity to HAV, for which only one serotype has been described. Temporary protection against hepatitis A for susceptible individuals can be achieved by injection of immune serum globulin, but at present there is no vaccine available.

The 27 nm virion of HAV was first visualized in 1973. See, Feinstone, S. M., Kapikian, A. Z. & Purcell, R. H. (1973) Science 182, 1026-1028. It was first isolated in tissue culture in 1979. See, Provost, P. J. & Hilleman, M. A. (1979) Proc. Soc. Exp. Biol. Med. 160, 213-221. Recently, HAV has been classified as a picornavirus. See, Coulepis, A. G., Locarnini, S. A., Westaway, E. G., Tannock, G. A. & Gust, I. D. (1982) Intervirology 18, 107-127.

HAV has a sedimentation coefficient of approximately 160 S and a primary buoyant density of 1.34 g ml in CsC1. Virion capsid polypeptides of $M_r$ = 32,000, 26,000, 22,000 and 10,000 have been described. The single-stranded infectious RNA has a molecular weight of about $2.5 \times 10^6$; various genome lengths (between 6700-8100 nucleotides) have been reported. It contains poly(A), presumably at the 3' terminus. By analogy with other picorna-viruses, the RNA should contain an open reading frame of about 6500 nucleotides which directs synthesis of a polyprotein that is post-translationally cleaved into virion proteins. These include the four capsid proteins, a peptide linked to the 5' end of the genome (VPg), an RNA-dependent RNA polymerase, and a protease. Putnak, J. R. & Phillips, B. A. (1981) Microbiol. Rev. 45 . 287-315; Kitamura, N., Semler, B. L., Rothberg, P. G., Larsen, G. R., Adler, C. J., Dorner, A. J., Emini. E. A., Hanecak, R., Lee, J J., van der Werf, S., Anderson, C. W. & Wimmer, E. (1981) Nature (London) 291. 547-553; Racaniello, V. R. & Baltimore, D. (1981) Proc. Natl. Acad. Sci. USA 78 , 4887-4891; and. Nomoto,-A.. Omata, T., Toyoda, H., Kuge, S., Horie, H., Kataoka, Y., Genba, Y., Nakano, Y. & Imura, N. (1982) Proc. Natl. Acad. Sci. USA 79, 5793-5797.

Despite extensive efforts, analysis of the molecular structure and events in replication of HAV have been impeded because of the difficulty in cultivating HAV in cell culture. Although attempts have been made to clone cDNA for HAV, these have met with only limited success. For example, cloned cDNAs of less than 1,000 base pairs have been reported. Von der Helm, K., Winnacker, E. L., Dienhardt, F., Frosner, G. G., Gauss-Muller, V., Bayerl, B., Scheid, R. and Siegl, G. J. Virol Methods 3: 37-43 (1981).

Disclosure of the Invention

The present invention relates to clonal cell lines transformed with particular recombinant DNA vectors. More specifically, the invention provides a clonal cell line transformed with a recombinant DNA vector containing any one of $HAV_{LB}$ 58 (ATCC 39454), $HAV_{LB}$ 113 (ATCC 39455), $HAV_{LB}$ 148 (ATCC 39456), $HAV_{LB}$ 207 (ATCC 39457), $HAV_{LB}$ 228 (ATCC 39458) and $HAV_L$ 1307 (ATCC 39459).

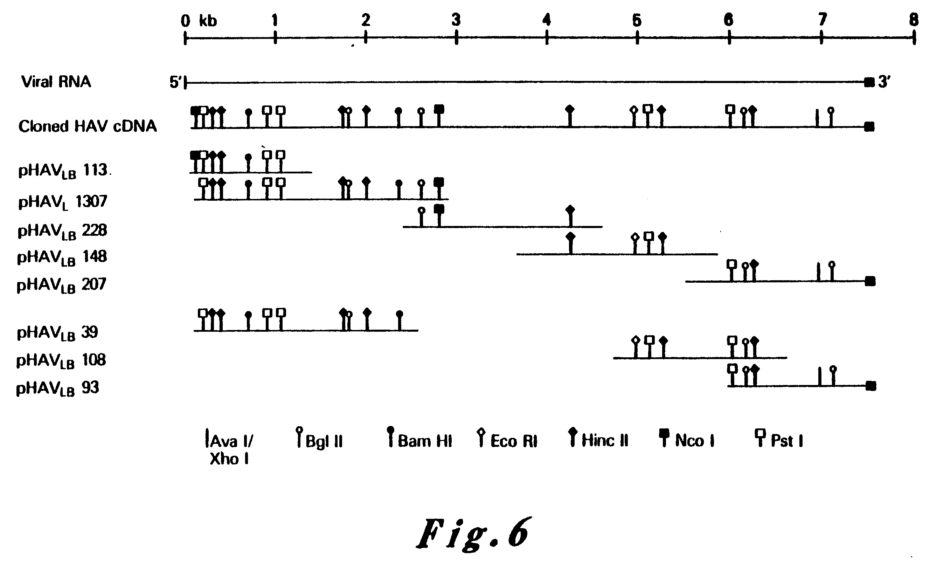

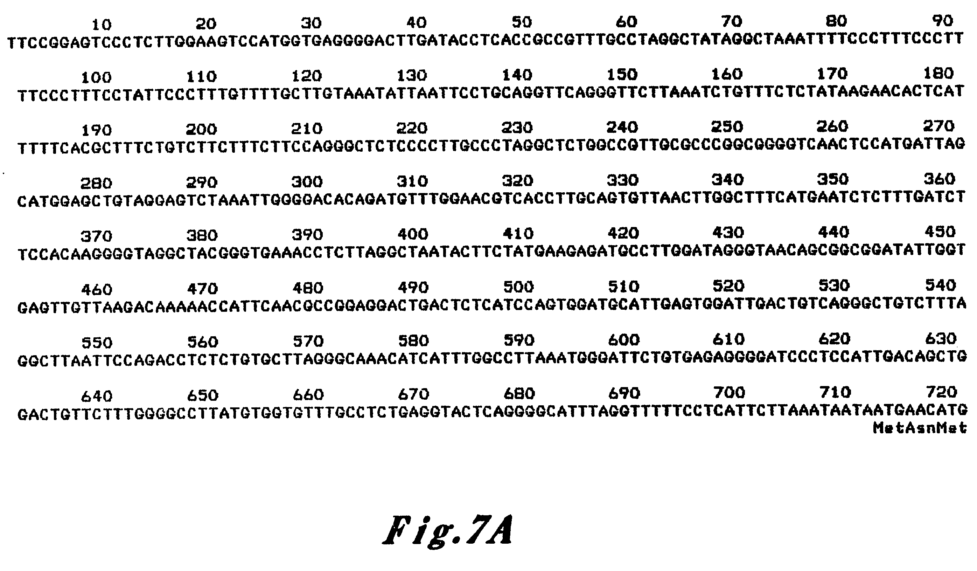

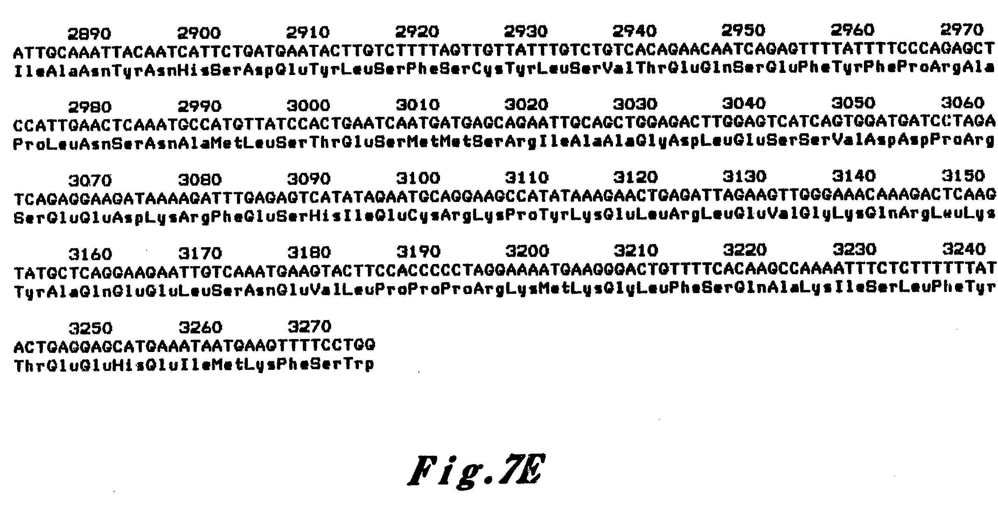

In one embodiment, the invention provides a clonal cell line transformed with a recombinant DNA vector containing $HAV_{LB}$ 113 consisting essentially of those nucleotides of Figures 7A and $7_B$ below which correspond to the respective lengths shown in the restriction maps of Figure 6 below.

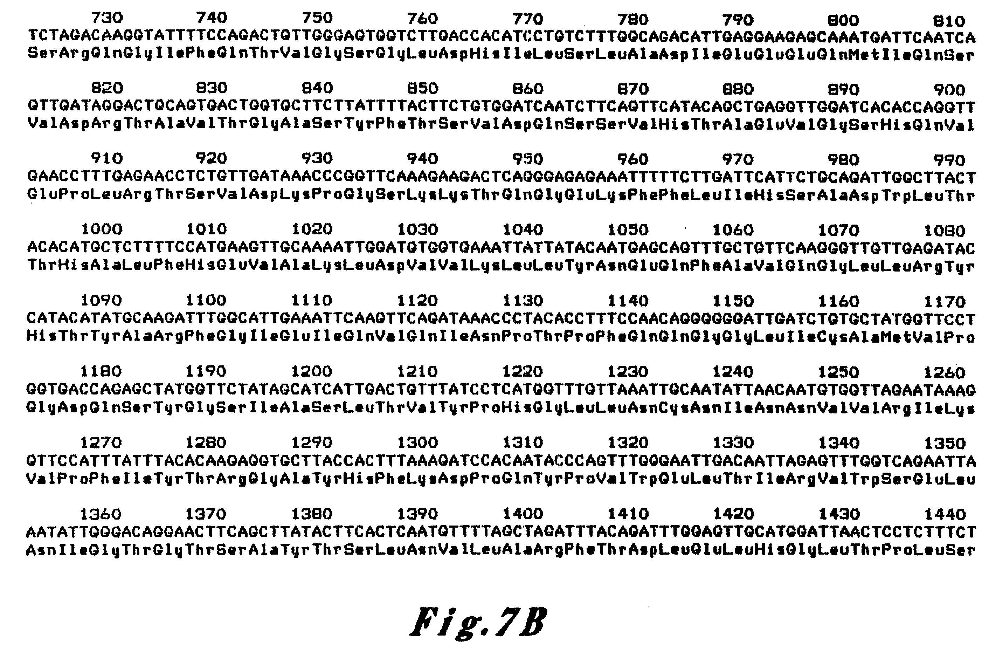

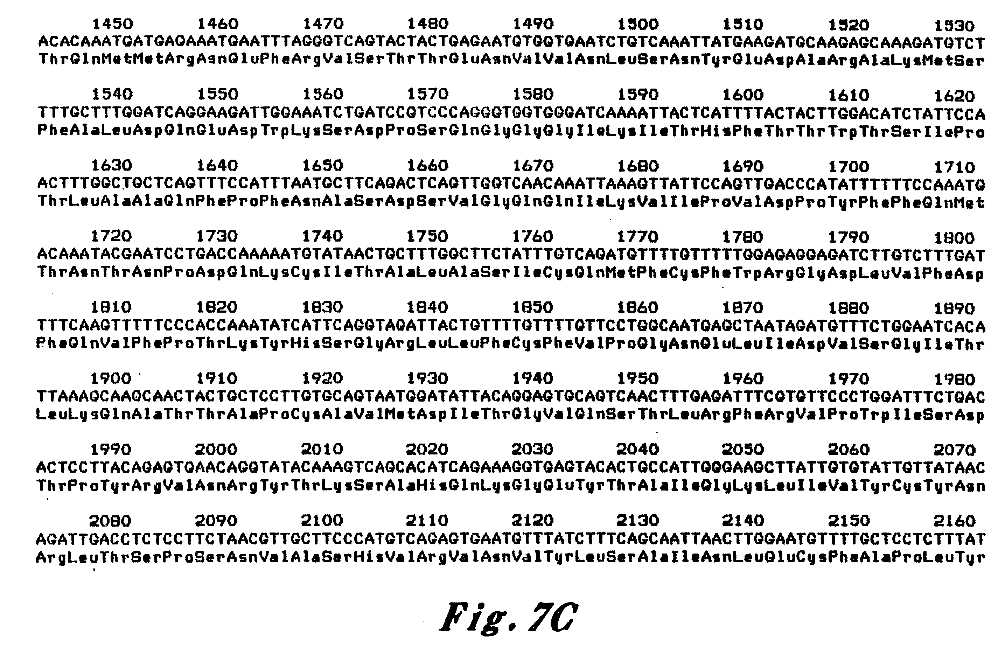

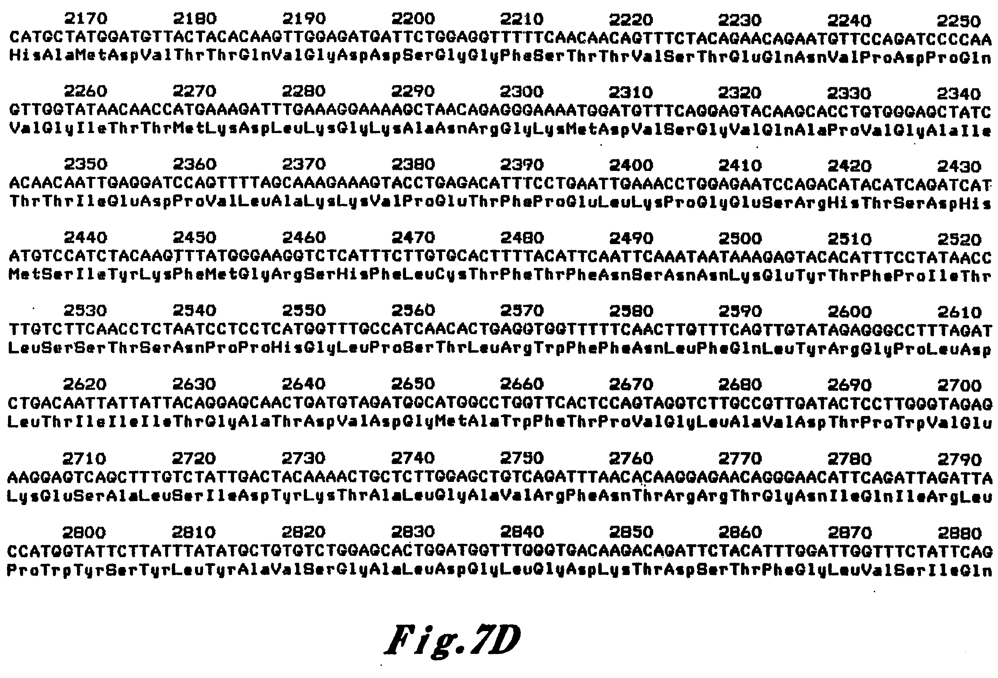

In another embodiment, the invention provides a clonal cell line transformed with a recombinant DNA vector containing $HAV_L$ 1307 consisting essentially of those nucleotides of Figures 7A, 7B, 7C, 7D and 7E below which correspond to the respective lengths shown in the restriction maps of figure 6 below.

The present invention also provides HAV cDNA derived from a clonal cell line as described hereinabove.

The HAV cDNA is produced by reverse transcribing HAV RNA and inserting the resulting cDNA molecule into a recombinant DNA vector to give a recombinant DNA vector of the type described hereinabove. Appropriate cells are then transformed with the recombinant DNA vector, cloned and grown under conditions sufficient for production of HAV cDNA. This cDNA can then be harvested from the clonal cell culture and used, as is, or further modified for certain applications.

In a particular embodiment, bacteria are modified by genetic engineering techniques to make such bacteria capable of producing HAV double-stranded complementary DNA (ds cDNA). In this method, HAV single-stranded (ss) RNA is reverse transcribed to provide HAV ss cDNA which is extended to ds cDNA and then inserted into a bacterial plasmid to create a chimeric plasmid containing the nucleotide sequences defined hereinabove. The chimeric plasmid containing the ds cDNA is then inserted into bacterial cells by transforming the bacterial cells with the chimeric plasmid. Bacterial cells which have been so transformed can then be cloned and clonal cell lines grown in cell culture to replicate the chimeric plasmid. The HAV ds cDNA can then be recovered by enzymatically cleaving it from replicated chimeric plasmids.

This method provides for the microbiological production of relatively large quantities of HAV cDNA at reasonable costs. The cDNA, in turn, can be employed in assays for the detection of HAV since HAV cDNA will bind specifically to HAV RNA. Such assays can be performed quickly and easily and they offer the potential for being extremely sensitive and specific for HAV virus detection.

In a further aspect therefore, the present invention provides an assay for the detection of HAV in a sample (eg. a sample selected from human physiological samples, laboratory animal physiology samples, environmental samples and tissue culture samples), comprising:

a) isolating an RNA-containing fraction of said sample, said RNA-containing fraction being a fraction which would contain HAV RNA if HAV were originally present in said sample;

b) labeling HAV cDNA obtained as described hereinabove;

c) incubating said labeled cDNA with said RNA-containing fraction in an incubation mixture under conditions sufficient for labeled cDNA to hybridise to HAV RNA;

d) removing unbound labeled cDNA from the incubation mixture; and

e) detecting remaining bound labeled cDNA.

HAV cDNA can also be employed in the production of either HAV antigen or antibodies to such an antigen. In these methods, HAV cDNA is produced as described above. For antigen production, HAV cDNA capable of directing antigen synthesis is selected and inserted into cells capable of producing the antigen after which the cells are cultured under conditions suitable for antigen production and harvesting. An alternative method of antigen production is the synthesis of peptides, in vitro. The amino acid sequences of such peptides can be deduced from the determined nucleotide sequence of cloned HAV cDNA. For antibody production, harvested antigen is employed to immunize a host capable of producing antibodies to HAV. Monoclonal antibodies can be produced employing antibody-producing cells from the host and known techniques, such as the formation of hybridoma cell lines.

Brief Desciption of the Drawings

FIGURE 1 is a schematic diagram illustrating the production of a bacterial chimeric plasmid containing HAV ds cDNA;

FIGURE 2 is a block diagram illustrating one embodiment of an assay employing HAV cDNA produced according to the methods described herein;

FIGURE 3 shows the results of an alkaline agarose gel electrophoresis of cDNA from HAV RNA and poliovirus type 2 RNA;

FIGURE 4 illustrates the results of alkaline agarose gel electrophoresis of products of preparative HAV ds-cDNA synthesis;

FIGURE 5 illustrates the results of hybridization of cloned cDNA insert probe to HAV RNA;

FIGURE 6 is a restriction map of HAV cDNA clones prepared according to this invention;

FIGURES 7A-7E illustrate the nucleotide sequence of cloned HAV cDNA and predicted amino acid sequence from near the genome 5' terminus to the end of the area corresponding to the capsid protein region of poliovirus RNA; and

FIGURES 8A and 8B illustrate the nucleotide sequence of cloned HAV cDNA and predicted amino acid sequence corresponding to the RNA polymerase region of poliovirus RNA and the genome 3' terminus.


Best Mode for Carrying Out the Invention

The methods described herein for producing HAV cDNA employ fundamental gene splicing techniques which have been described in the scientific literature. For example, U.S. Patent No. 4,227,224, issued to Stanley N. Cohen and Herbert W. Boyer, on December 2, 1980, describes many of these techniques. The teachings of the Cohen and Boyer patent, therefore, are incorporated herein by reference.

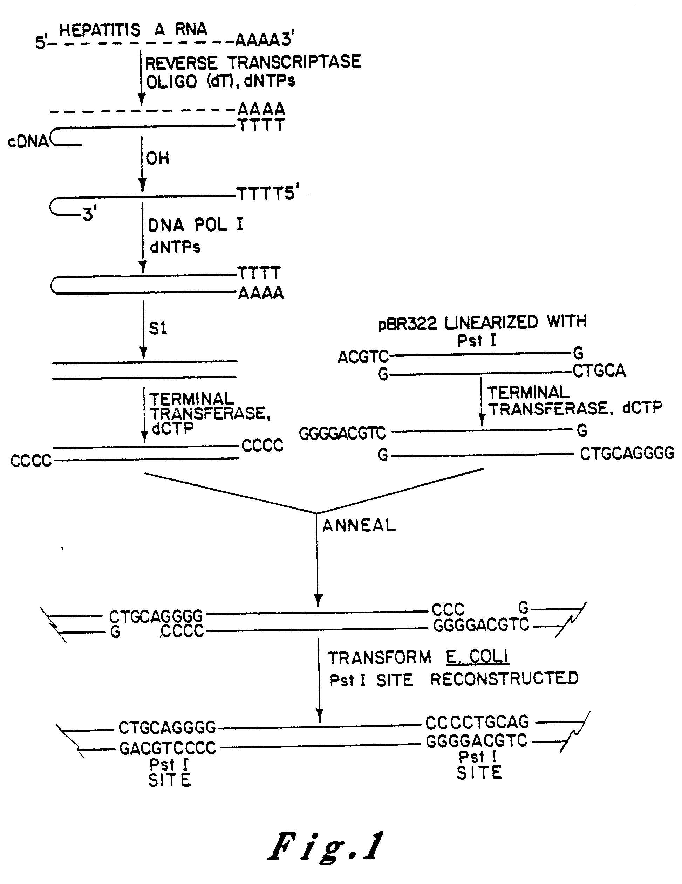

A more specific description of the techniques which can be employed in producing HAV ds cDNA will now be presented in conjunction with Figure 1, a schematic diagram illustrating these techniques.

HAV was isolated from human stool during a family outbreak in Australia and passaged twice in marmosets. This virus, present in a homogenate of liver tissue, was injected intravenously into marmosets that were monitored for HAV production by immunofluorescent antibody analysis of biopsied liver tissue. The animals were killed when maximum immunofluorescence was reached, at which time their livers were removed, minced, frozen, and subsequently homogenized prior to purification of HAV. Alternatively, HAV can be purified directly from homogenates of human stool or HAV-infected tissue culture cells.

When isolated from liver or stool, the bulk of extraneous tissue and debris can be removed by low speed centrifugation. Disruption of cellular membranes that may bind or contain HAV can be accomplished by extensive hydrocarbon extraction. Micrococcal nuclease can be used to digest and eliminate non-encapsidated nucleic acids that might ultimately contaminate purified HAV RNA. The virus can be purified using successive steps of rate zonal and isopycnic density gradient centrifugation. Those skilled in the art will recognize that there are methods of purifying HAV that can be used alternatively or in conjunction with those above, such as gel filtration chromatography or ion-exchange chromatography.

HAV ss RNA can be extracted from the purified viral particles by digestion with sodium dodecyl sulfate (NaDodSO₄) and proteinase K, followed by extraction with phenol and chloroform isoamyl alcohol. HAV ss RNA is then employed in the synthesis of HAV ds cDNA, as illustrated. Initially, the HAV ss RNA is reverse transcribed employing the enzyme reverse transcriptase, also known as RNA-dependent DNA polymerase. See Kacian D.L. and Myers, J.C. (1976) PNAS 73 :2191-5. Typically, Tris-HCl buffer (pH 8.3), magnesium ions (mg⁺⁺), potassium ions, dithioerythritol, sodium pyrophosphate, a ribonuclease inhibitor (RNasin), the four deoxynucleoside triphosphates (dATP, dCTP, dGTP and TTP), and at least one labeled deoxynucleoside triphosphate for monitoring the product are added to the reaction mixture. Oligo(dT) is also added as a primer which hybridizes to the poly(A) end of HAV RNA thereby providing a site for initiation of reverse transcription. The reaction mixture is incubated under conditions to allow the enzyme to synthesize a complementary ss DNA copy of the HAV genome starting from the 3' poly(A) end and continuing to the 5' end of the genome.

The RNA template is then removed with alkali. The cDNA molecules are then placed in another reaction mixture containing Tris-HCl buffer (pH 7.5), Mg⁺⁺, dithiothreitol, the 4-deoxynucleoside triphosphates, and the Klenow fragment of DNA polymerase I. This reaction mixture is maintained under conditions sufficient to allow the DNA polymerase I to extend the cDNA molecule, initiating synthesis at the snap-back formed at the 3' end of the molecule. In a typical example, the reaction mixture might be incubated at 37° for about 30 minutes, which is usually sufficient for formation of the second complementary DNA strand, as illustrated. See Humphries et al. (1978), Nucleic Acids Res., 5 :905-24.

S1 nuclease is then employed to cleave the loop at one end of the molecule. See Bhat and Piatigorsky (1975), PNAS, 76 :3299-3303.

The ds cDNA can then be tailed with oligo(dC) at the 3' ends by employing terminal transferase and dCTP. See Boyer et al. (1977) in "Recombinant Molecules: Impact on Science and Society" (R. F. Beers and E. G. Bassett, eds.) pp 9-20, Raven, N. Y. This produces ds cDNA having poly(C) at both ends to serve as "sticky" ends in subsequent binding to a cleaved bacterial plasmid in order to form a recombinant DNA molecule. The tailed ds DNA can be filtered by gel chromatography, and the largest fragments are then pooled from the eluted fractions.

Plasmid pBR322 can be employed to illustrate chimeric plasmid formation. Plasmid pBR322 is a well characterized plasmid known to contain selectable markers. This plasmid contains one gene coding for tetracycline resistance as well as a gene coding for ampicillin resistance. Since the HAV ds cDNA sequences are inserted into the gene for ampicillin resistance, successfully transformed bacterial cells are ampicillin sensititve (Amp$^s$) and tetracycline resistant (Tet$^R$), the latter providing a marker for transformed cells.

Plasmid pBR322 is cleaved using the restriction enzyme Pst I at the gene coding for ampicillin resistance. The resulting linearized plasmid is then tailed with oligo(dG) employing the enzyme terminal transferase and dGTP to produce "sticky" ends on the linearized cleaved plasmid chains.

The oligo(dG) tailed plasmid DNA and the oligo(dC) tailed HAV ds cDNA are than hybridized in solution. This can be accomplished by mixing these DNA species in an equimolar ratio in 0.1 M NaCl, heating for 3 minutes at 68° and then incubating at 42° for 2 hours. See Boyer, H.W., Bettlach, M., Bolivar, S. F. Rodriguez, R. L., Heyneker, H. L., Shine, J. and Goodman, H. M., (1977), "Recombinant Molecules: The Construction of Molecular Cloning Vehicles," in Recombinant Molecules: Impact On Science and Society, eds. Beers, R. F. and Bassett, E. C. pp. 9-20, Raven Press, N.Y.

The hybridized plasmid-HAV ds cDNA is then inserted into E. coli in order to reconstruct the Pst I site, to amplify the plasmid DNA, and to identify clones which contain recombinant plasmids. See Dagert, M. and Ehrlich, S. D. (1979), Gene, 23-28. Once the hybrid molecule is inserted, the single-stranded gap is repaired by the bacteria. This reconstruction provides two Pst I sites which the Pst I enzyme can later recognize and cleave to separate the HAV ds cDNA sequences from replicated plasmids.

E. coli cells transformed with the hybrid molecules can then be selected in the presence of tetracycline and later screened for ampicillin sensitivity. Those clones identified as Tet$^r$ Amp$^s$ can then be analyzed by using well-known techniques to obtain recombinant plasmids which are cleaved with Pst I and sized by gel electrophoresis. Comparison of the electrophoretic pattern of digested hybrid plasmids with marker DNA fragments of known lengths indicates the lengths of the inserted cDNAs.

To establish the identity of a cloned cDNA, it can be hybridized to RNA from cells, including cells infected with HAV. Inserted cDNA can be isolated from plasmids by digestion with Pst I and electrophoresis in low melting point agarose. See, Cummings, I.W., et al., Proc. Natl. Acad. Sci. USA 77 :1842-1846 (1980). The agarose gel containing the cDNA is melted and the cDNA recovered by extraction with phenol and with chloroform isoamyl alcohol and then by precipitation with ethanol. Inserted cDNA can then be labeled with $^{32}$P by nick translation. See, Rigby, P.W.J., et al. J. Molec. Biol. 113: 237-251(1977). RNA can be isolated from cells, for example, by lysis with detergents, extraction with phenol and chloroform isoamyl alcohol, and precipitation with ethanol. See Maniatis, T., et al., Molecular Cloning, Cold Spring Harbor Laboratory, Cold Spring Harbor, N.Y., 1982. This RNA can then be electrophoretically separated in agarose and transferred to nitrocellulose paper. See, Thomas, P.S., Proc. Natl. Acad. Sci USA 77 :5201-5205 (1980). The RNA on the nitrocellulose paper is hybridized with $^{32}$P-labeled cDNA insert in a sealed polyethylene bag. Autoradiography of the washed and dried paper reveals which RNA species the cloned cDNA species is derived from, since the labeled cDNA will hybridize to the RNA and appear as dark band(s) on the X-ray film which is exposed to the nitrocellulose paper. When the cloned cDNA species contains specific HAV sequences, the predominant RNA species identified will have the characteristic genomic length of picornaviral RNA (approximately 7500 nucleotides) and will be found only in RNA from HAV-infected cells. Direct screening of clones by colony hybrdization is avoided since any probe prepared directly from purified HAV (labeled RNA or cDNA) might be as contaminated with nonviral sequences as the RNA template used for cloning.

Those skilled in the art will recognize, of course, that other materials and conditions can be employed other than those specifically described in the aforementioned embodiments. For example, it is clear that bacterial cells other than E. coli could be employed. For example, B. subtilis could also be employed as well as many other bacterial strains.

Similarly, although bacterial plasmids have been employed in producing HAV cDNA sequences, other recombinant DNA vectors could be employed. Examples of other recombinant DNA vectors include phages, animal viruses and yeast vectors. Hosts which allow the recombinant DNA vector to multiply are chosen, of course.

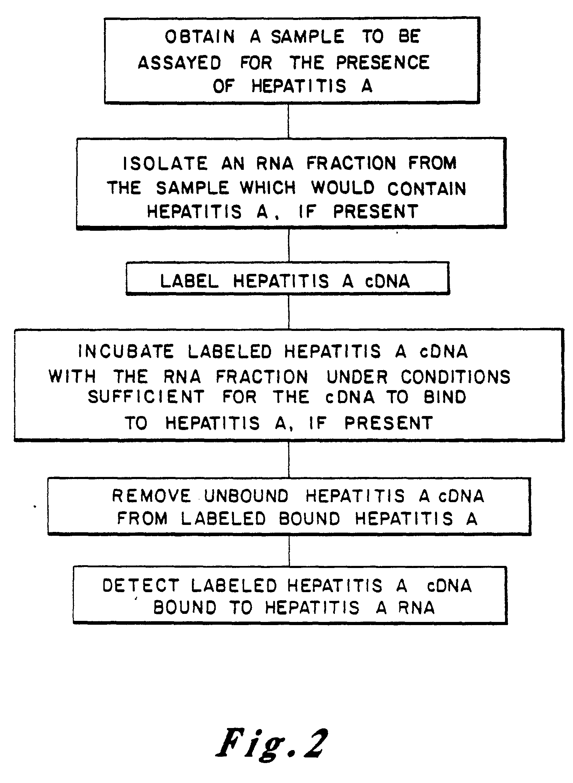

One significant use for HAV cDNA produced according to this invention is in assays to detect the presence of HAV RNA. In a typical assay for HAV, for example, a patient sample, such as a stool specimen, can be assayed as illustrated in Figure 2. The RNA fraction of the patient sample is first isolated, which can be done by phenol extraction and ethanol precipitation. This RNA fraction need not be pure, but it must be a fraction which would contain HAV RNA if HAV were present in the original sample. HAV cDNA is first labeled, e.g., with a radioactive material such as tritium, iodine, or $^{32}$P, and subsequently incubated with the RNA fraction under conditions to allow the labeled HAV cDNA to bind to HAV RNA, if present. After incubation, unbound labeled HAV cDNA is separated and bound labeled HAV cDNA is then detected in a scintillation counter or by other means.

Other patient samples, of course, such as a biopsy, might be employed. Additionally, the assay can be performed on other samples which might contain HAV, such as sewerage, water or shellfish suspected to be contaminated.

A solid-phase assay, although not illustrated, might be performed. Additionally, the label need not be a radioactive isotope, but might be an enzyme, optical label, etc.

In particular, it is believed that "dot blots" of stool extracts hybridized with HAV cDNA probe will provide a sensitive and easily performed assay for the presence of HAV. Such "dot blots" have been described. See Thomas, PNAS 77, 5201-5205 (1980).

Research laboratories studying HAV could employ HAV cDNA probes for detecting or quantitating HAV RNA in specimens such as cells or fluid from tissue culture, tissues and stool from experimentally infected primates, and specimens collected from patients. In the case of intact cells, the assay might be an in situ hybridization.

Another significant use for HAV cDNA produced according to this invention is in the production of HAV antigens. Antigenic proteins can be produced by reverse transcribing HAV RNA to provide cDNA, inserting the cDNA into a recombinant DNA vector and transforming cells in which said recombinant DNA vector can multiply. Transformed cells can then be cloned to produce a cell line capable of replicating the cDNA. The cell line can be cultured under conditions sufficient for the production of cDNA and cDNA can then be harvested from the cell culture. Specific cDNA could be selected and isolated which was capable of directing antigen synthesis in prokaryotic or eukaryotic cells and subsequently inserted into such cells so that these cells produce antigen. Alternatively, antigenic peptides can be synthesized in vitro. Cloned cDNA is produced and harvested as described above and its nucleotide sequence is determined. The amino acid sequences of such peptides are deduced from the nucleotide sequence of appropriate regions of the HAV genome (e.g., that coding for the capsid proteins). HAV antigen produced either by cells or in vitro could be used as an alternative source of the antigen component in the currently available immunoassays for anti-HAV in serum.

Antigen produced by the methods described above could be used to immunize a host, such as an animal, and cause that host to produce antibodies against HAV or a portion thereof. If desired, antibody producing cells could be employed to produce cell lines capable of producing monoclonal antibodies. Polyclonal or monoclonal antibodies would be useful reagents for laboratories involved in the study of HAV. Also, harvested antigen capable of eliciting protective anti-HAV in humans could be an effective means of vaccination. For example, a capsid protein produced from a suitable cell could possess sufficient antigenic properties for use in a vaccine against HAV, as has been demonstrated for a strain of foot and mouth disease virus (FMDV), another member of the picornavirus group. See, Kleid, D.G., et al. Science 214 :1125-1129 (1981). Neutralizing antibodies against FMDV have also been elicited using synthetic peptides as antigens. See, Bittle, J.L., et al., Nature (London) 298 :30-33 (1982).

The invention is further and more specifically illustrated by the following examples.

EXAMPLE 1

PREPARATION OF HYBRIDIZED PLASMID - HAV DS CDNA AND CLONING IN E. COLI

Preparation of HAV.

The HM-175 strain of HAV was isolated from a family outbreak in Australia. See, Daemer, R. J.,

Feinstone, S. M., Gust, I. D. & Purcell, R. H. (1981) Infect. Immun. 32 , 388-393. HAV which had been passaged twice in marmosets (Saguinus mystax and S . labiatus) was inoculated into eight marmosets. A 20% wgt/vol suspension of HAV-infected liver in phosphate buffered saline was injected intravenously and animals were monitored by serum enzymes and immunofluorescence of liver biopsy. See, Mathiesen, L. R., Feinstone, S. M., Purcell, R. H. & Wagner, J. (1977) Infect. Immun. 18 , 524-530. When maximum immunofluorescence was reached 10-14 days after inoculation, the marmosets were killed and their livers immediately removed, minced, frozen in liquid $N_2$, and stored at -70° C until virus purification.

HAV was also passaged 6 times in marmosets and 19 times in secondary African green monkey kidney (AGMK) monolayers which were used as a source of RNA for hybridization. Infected cultures were harvested at 21 days when 100% of the cells exhibited maximal immunofluorescence.

Virus Purification. Minced marmoset livers were homogenized (40% wgt/wgt) in phosphate-buffered saline (PBS), 0.1% NP-40 in a Sorvall Omnimixer on ice. Particulate matter was removed by centrifugation at 13,000 g (pellets were re-extracted with PBS, 0.1% NP-40 and with trichlorotrifluoroethane/ chloroform). The homogenate was concentrated in a Beckman Type 45Ti rotor at 40,000 rpm for 16 hr at 5° C. Pellets were resuspended in 10 mM Tris-HCl (pH 7.5), 0.1 M NaCl, 0.1% NP-40 (TNN) and extensively extracted with trichlorotrifluoroethane and chloroform. Pooled aqueous layers were again concentrated in a Beckman Type 45Ti rotor at 40,000 rpm for 16 hr at 5° C and pellets resuspended in 20 ml of TNN. The virus suspension was then treated with micrococcal nuclease (75 units/ml) in TNN, 1mM $CaCl_2$ for 15 min at 20° C. The reaction was stopped by the addition of EGTA (ethylene glycol-bis[ -aminoethyl ether] N,N,N',N'-tetraacetic acid) to 2 mM. See, Pelham, H.R.B. et al., Eur. J. Biochem. 67 :247-256(1976). This preparation was centrifuged over an 8 ml cushion of 30% (wgt/wgt) sucrose in TNN in a Beckman SW27 rotor at 25,000 rpm for 16 hr at 5° C. The resulting pellet was resuspended in 0.8 ml TNN and layered over a 20-40% (wgt/wgt) gradient of sucrose in TNN, centrifuged in a Beckman SW27 rotor at 25,000 rpm for 4 hr at 5° C, and separated into 1 ml fractions. Those fractions with high reactivity in a solid phase radioimmunoassay (SPRIA) for HAV were pooled with solid CsCl added to a density of 1.36 g/ml and centrifuged in a Beckman Type 75Ti rotor at 60,000 rpm for 40 hr at 18° C. See, Purcell, R.H. et al., J. Immunol. 116 :349-356(1976). Fractions with HAV by SPRIA were pelleted in a Beckman SW27 rotor for 17 hr at 5° C. The pellet was resuspended in 0.7 ml TNN and layered over a sucrose gradient as described above. The HAV-containing fractions were dialyzed against 10 mM Tris-CHI (pH 7.5), 0.1 M NaCl, 1 mM ethylene diamine tetraacetic acid (EDTA). Direct electron microscopic examination of purified HAV particles revealed a homogeneous population of 27 nm virions.

RNA Extraction and Characterization. Suspensions of purified HAV were incubated at 37° for 15 min with 500 ug/ml proteinase K, after which NaDodSO₄ was added to a concentration of 0.5% wgt vol and incubation was continued for an additional 30 min. After extraction with phenol and then with chloroform-isoamyl alcohol (24 vol:1 vol), RNA was precipitated in ethanol, redissolved and, after removing a portion for analysis, reprecipitated. RNA was characterized by UV spectroscopy and agarose gel electrophoresis after denaturation with 1 M glyoxal and 50% (vol vol) dimethysulfoxide. See, McMaster, G. K. & Carmichael, G. G. (1977) Proc. Natl. Acad. Sci. USA 74, 4835-4838. HAV RNA contained a discrete band comigrating with poliovirus type 1 RNA (approximately 7440 nucleotides). See Kitamura, N., Semler. B. L., Rothberg, P. G., Larsen. G. R.. Adler. C. J.. Dorner, A. J.. Emini. E. A.. Hanecak, R., Lee, J. J., van der Werf, S., Anderson, C. W. & Wimmer. E. (1981) Nature (London) 291, 547-553; Racaniello, V. R. & Baltimore. D. (1981) Proc. Natl. Acad. Sci. USA 78 , 4887-4891; and Nomoto, A.. Omata, T., Toyoda, H., Kuge, S., Horie, H., Kataoka, Y., Genba, Y., Nakano, Y. & Imura, N. (1982) Proc. Natl. Acad. Sci. USA 79 , 5793-5797. Other portions were used to analyze template quality and optimal conditions for cDNA synthesis.

RNA for hybridization studies was prepared from uninfected and HAV-infected AGMK cells by isolation of total cytoplasmic RNA. See, Maniatis, T., Fritsch, E. F. & Sambrook, J. (1982) Molecular Cloning (Cold Spring Harbor Laboratory, Cold Spring Harbor, N.Y.). Polyadenylated RNA was isolated by oligo(dT)-cellulose chromatography. See, Varmus, H. E., Quintrell, N. & Ortiz, S. (1981) Cell 25 , 23-36. RNA was transferred to nitrocellulose paper after electrophoretic separation through agarose gels containing glyoxal-dimethylsulfoxide denatured RNA. See, Thomas, P. S. (1980) Proc. Natl. Acad. Sci. USA 77, 5201-5205.

The yield of virion RNA from eight marmoset livers was approximately 1.0 ug and the $A_{260} \cdot A_{280}$ ratio was 2.0.

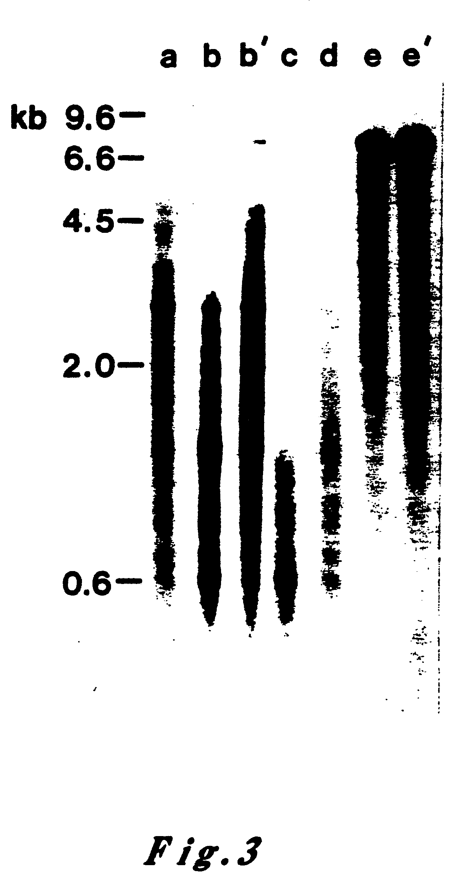

Preparation of cDNA Clones. RNAs from several sources of HAV and from poliovirus type 2 were compared for template quality in cDNA synthesis using alkaline agarose gel electrophoresis. RNAs (5 ug ml or less) were incubated for 60 min at 42.5° C in 10 ul containing 50 mM Tris-HCl (pH 8.3); 10 mM MgCl₂: 50 mM KCl; 500 uM each: dATP. [ ³²P]dCTP (2 Ci mmol), dGTP, and TTP; 0.4 mM dithioerythritol; 4 mM sodium pyrophosphate; 30 ug ml oligo (dT₁₂₋₁₈); and 80 units ml reverse transcriptase. Two reactions (cDNAs in Fig. 3, lanes b' and e') also contained 2000 units/ml RNasin. The resulting reverse transcripts from RNA templates are illustrated as follows in Fig. 3: lane a, HAV RNA derived from marmoset liver; lanes b and b', HAV RNA from AGMK cells; lane c, HAV RNA from human stool; lane d, HAV RNA from AGMK cells; lanes e and e', poliovirus type 2 RNA. HAV RNAs were isolated as described above except that derived from human stool (lane c) which was extracrted from purified virus (a gift from S. Locarnini) and an earlier preparation from AGMK cells (lane d) using modifications of standard procedures. Migration of <u>Hind</u> III fragments in kilobases (kb) is indicated to the left of the figure.

HAV RNA derived from liver or AGMK culture yielded a series of transcripts ranging in size from slightly smaller than the longest poliovirus type 2 cDNA to less than 500 nucleotides (Fig. 3, lanes a, b, and b'; lanes e and e' show poliovirus type 2 cDNA). Presumably, degradation of HAV RNA prevented more extensive synthesis of reverse transcript approaching the expected full length of 7500 nucleotides. Other HAV cDNAs in Fig. 3 (lanes c and d) revealed evidence of more extensive RNA degradation, but all gave a similar banding pattern which was different from that of poliovirus cDNAs.

The effect of varying several chemical constituents and physical parameters on yield and length of HAV cDNA was analyzed by alkaline agarose gel electrophoresis and incorporation of ($^{32}$P]dCMP into trichloroacetic acid-precipitable product. Incubation for 30 min, with 120 units/ml reverse transcriptase, or with 100 mM KCl increased size and quantity of cDNA when compared to that shown in Fig. 3, lane b'. Denaturation of HAV RNA was not attempted because quantity was limited and, in earlier experiments, heat or methyl mercury treatment of poliovirus RNA decreased yield and size of cDNA.

Preparative conditions for HAV cDNA synthesis were based on findings in the analytical experiments described above. HAV RNA (0.8 ug) derived from marmoset liver served as a template for cDNA synthesis using reverse transcriptase (120 units/ml) for 30 min at 42.5° C in 160 ul that contained 50 mM Tris-HCl (pK 8.3); 10 mM MGCl$_2$; 100 mH KCl; 500 uM each: dATP, [ $^{32}$P]dCTP (0.025 Ci/mmol), dGTP, and TTP; 1 mM dithioerythritol; 4 mM sodium pyrophosphate; 30 ug/ml oligo(dT$_{12-18}$); and 2000 units/ml RNasin. After addition of EDTA to 20 mM, RNA-cDNA hybrids were isolated by phenol extraction, column chromatography and ethanol precipitation. The RNA template was hydrolyzed in 0.3 N NaOH, 0.7 M NaCl and 5 mM EDTA for 2 hr at 37° C.

The second strand of cDNA was synthesized for 30 min at 37° C using the large (Klenow) fragment of E. coli DNA polymerase I (28 units/ml) in 10 mM Tris-HCl (pH 7.5) ; 5 mM MgCl$_2$; 5 mM dithioerythritol; 50 uM each: dATP, [ $^{32}$P]dCTP (0.45 Ci/mmol), dGTP, and TTP; and cDNA (1 ug/ml).

After phenol extraction, column chromatography, and ethanol precipitation, double-stranded cDNA (ds-cDNA) was digested for 1 hr at 37° C using 10 units/ml nuclease S1 (0.1 units of S1 per ng ds-cDNA) in 30 mM NaOAc (pH 4.5), 0.3 M NaCl, 3 mM ZnCl$_2$, and 5% vol/vol glycerol, followed by addition of EDTA to 35 mM, phenol and ether extraction, and dialysis against 10 mM Tris-HCl (pH 7.5) and 1 mM EDTA.

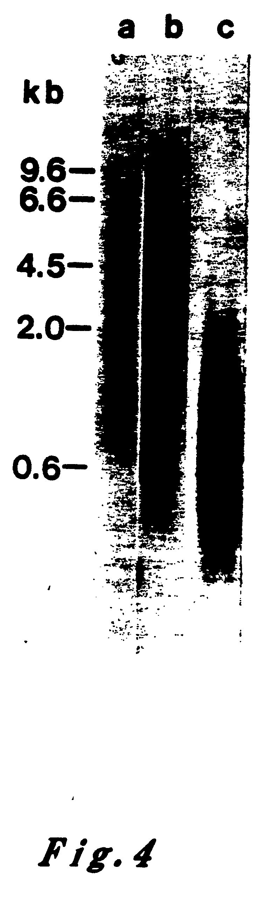

Products of preparative HAV ds-cDNA synthesis were analyzed by alkaline agarose gel electrophoresis and the results are shown in Fig. 4: lane a, approximately 3 ng cDNA; lane b, approximately 2 ng ds-cDNA prior to nuclease S1 digestion; lane c, approximately 2 ng ds-cDNA after nuclease S1 digestion. Migration of Hind III fragments in kilobases (kb) is indicated to the left of the figure. A significant portion of HAV cDNA transcribed under preparative conditions was 3000-7500 nucleotides in length (Fig. 4, lane a). However, a wide size range of ds-cDNA molecules was produced (Fig. 4, lane b) and most of the preparation was less than 2000 nucleotides in length after S1 digestion (lane c).

Homopolymer tails of dCMP were added to ds-cDNA using 250 units/ml terminal deoxynucleotidyl transferase for 20 minutes at room temperature in 100 ul that contained 0.14 M potassium cacodylate (pH 7.2), 1 mM CoCl$_2$ 0.2 mM dithioerythritol, 500 ug/ml nuclease-free bovine serum albumin, and 200 uM dCTP. After phenol extraction, 50% of ds-cDNA was ether-extracted and precipitated with ethanol.

The remaining tailed ds-cDNA was applied to a 3 ml column of Sepharose 4B in 20 mM Tris-HCl (pH 8.0), 0.6 M NaCl and 2 mM EDTA. See, Maniatis, T., Fritsch, E. F. & Sambrook, J. (1982) <u>Molecular Cloning</u> (Cold Spring Harbor Laboratory, Cold Spring Habor, N.Y.). The first five 65 ul fractions containing ds-cDNA were pooled and precipitated in ethanol after the addition of 2 ug yeast tRNA.

Plasmid vector pBR322, cleaved at the Pst I site and tailed with dGMP, was annealed to equimolar amounts of both tailed ds-cDNA preparations and used to transform E . coli HB101 by standard procedures. See, Maniatis, T. et al., Molecular Cloning Cold Spring Harbor Laboratory, Cold Spring Harbor, NY, 1982.

Analysis of cDNA Clones. Clones containing putative HAV sequences were screened by cleaving recombinant plasmid preparations with Pst I and sizing by gel electrophoresis. Approximately 200 ng ds-cDNA was synthesized. Ten ng of ds-cDNA selected for large size by gel filtration yielded 232 tetracycline-resistant E. coli transformants. Cleavage with Pst 1 demonstrated inserts of 1000 base pairs or greater in 43 of the 232 recombinant plasmids, designated pHAV$_{LB}$. An additional 2710 clones were obtained from 9 ng of unfractionated ds-cDNA. From this group only pHAV$_L$ 1307 (described below) was extensively characterized.

Cloned cDNA inserts isolated from low melting point agarose were labelled by nick translation and used as probes in hybridization (i) to electrophoretically-separated RNA bound to nitrocellulose paper (described above) for establishing the identity of cloned cDNA species, (ii) to DNA bound to nitrocellulose paper after lysis of bacterial colonies in situ for further screening, and (iii) to restriction fragments of DNA resolved by electrophoresis and bound to nitrocellulose paper for confirmation of tentative restriction maps constructed on the basis of single and double enzyme digests.

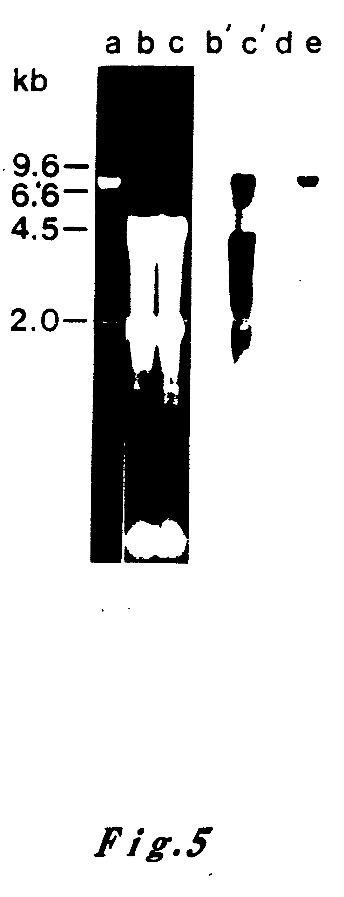

The identity of inserted DNAs in recombinant plasmids was established by hybridization to RNA bound to nitrocellulose paper after gel electrophoresis and the results are shown in Fig. 5. Nucleic acids were denatured and electrophoresed; marker lanes (a-c) were removed and stained with 2 ug/ml ethidium bromide in 0.05 N NaOH for 40 min; followed by 0.5 ug/ml ethidium bromide in 40 mM Tris-HOAc (pH 7.8), 1 mM EDTA for 15 min and destaining in the same buffer for 15 min. Lanes b'-e were transferred to nitrocellulose paper and hybridized for 36 hr at 42°C with 25 ng/ml [ $^{31}$P]pHAV$_{LB}$ 39 insert (5x10$^8$ dpm/ug) in 50% vol/vol formamide; 0.75 M NaCl; 0.075 M trisodium citrate; 50 mM sodium phosphate (pH 6.5); 0.2% NaDoSO$_4$; 100 ug/ml denatured sheared salmon sperm DNA; and 0.04% wgt/vol each: bovine serum albumin, polyvinyl-pyrollidone and Ficoll. Lane a contained 400 ng poliovirus type 2 RNA. Lanes b, b' and d contained cytoplasmic RNA from uninfected AGMK cells: lane b, 5 ug; lane b', 20 ug; and lane d, 1.5 ug, oligo(dT)-selected. Lanes c, c', and e contained cytoplasmic RNA from HAV-infected AGMK cells: lane c, 5 ug; lane c', 20 ug; and lane e, 1.5 ug, oligo(dT)-selected. Autoradiographic exposure for lanes b' and c' was one-eighth of that for lanes d and e. Migration of Hind III fragments in kilobases (kb) is indicated in Fig. 5.

A nick translated probe prepared from the insert of pHAV$_{LB}$ 39 specifically hybridized to RNA from HAV-infected AGMK cells (Fig. 5, lanes c' and e). Similar results were obtained when the inserts of pHAV$_{LB}$ 93 or pHAV$_{LB}$ 228 were used as probes. The predominant band identified had the size expected for genomic HAV RNA and comigrated with poliovirus type 2 RNA. Diffuse hybridization to lanes containing RNA from infected cells was probably due to RNA degradation. Nick translated pBR322 did not hybridize to any RNA species from either HAV-infected or uninfected AGMK cells, thereby eliminating the possibility that a small amount of pBR322 contaminating the insert probes was responsible for specific hybridization. None of the pHAV$_{LB}$ probes tested to date hybridized with poliovirus RNA.

The restriction map shown in Fig. 6 is based on data obtained from digests and on hybridization of labelled inserts to fractionated DNA. Viral RNA is estimated to be 7450 nucleotides in length excluding 3' poly(A) which is shown at the right (thicker line). A composite map of cloned HAV cDNA and positions of the inserts from clones pHAV$_{LB}$ 113, pHAV$_L$ 1307, pHAV$_{LB}$ 228, pHAV$_{LB}$ 148, and pHAV$_{LB}$ 207 are shown immediately below the viral RNA. Recombinant plasmids pHAV$_{LB}$ 39, pHAV$_{LB}$ 108, and pHAV$_{LB}$ 93 have been used for DNA sequencing, to confirm regions of overlap, or to prepare insert probes. Hybridization of an insert fragment from pHAV$_{LB}$ 228 (corresponding to the region from 2.4 to 3.0 kb, relative to scale) to DNA from bacterial colonies was used to select pHAV$_L$ 1307, pHAV$_{LB}$ 12, pHAV$_{LB}$ 58, and pHAV$_{LB}$ 153, for further analysis. Other plasmids which support this map include pHAV$_{LB}$ 153 (1:8-2.7 kb), pHAV$_{LB}$ 58 (1.9-4.0 kb), pHAV$_{LB}$ 12 (2.3-3.3 kb), pHAV$_{LB}$ 87 (4.1-5.8 kb), pHAV$_{LB}$ 201 (5.2-6.7 kb), pHAV$_{LB}$ 38 (5.2-6.9 kb), pHAV$_{LB}$ 56 (5.4-7.1 kb), pHAV$_{LB}$ 185 (5.5-7.5 kb), pHAV$_{LB}$ 24 (5.6-7.5 kb), and pHAV$_{LB}$ 122 (5.7-7.5 kb). Restriction sites have also been mapped with Acc I (2.0 kb), Apa I (5.7 and 6.2 kb), Ava II (3.5 and 6.6 kb), Bst EII (1.2 kb), Hind III (2.2 kb), Hpa I (0.4 kb) , Nde I (1.2 kb) , Pvu II (0.8, 1.0, 3.0, 5.2, 5.5, and 7.1 kb), Sac I (3.0 kb), and Xba I (0.8 kb). There is also a site for Nde I (2.5 kb) in pHAV$_{LB}$ 12, pHAV$_{LB}$ 58, pHAV$_{LB}$ 153, and pHAV$_{LB}$ 1307 which is not present in pHAV$_{LB}$ 39. There are no sites for Bgl I, Eco RY, Hae II, Kpn I, Mlu I, Nae I, Nar I, Nru I, Pvu I, Sac II, Sal I, Sma I, Sph I, Stu I, and Tth 111 I.

Plasmids pHAV$_{LB}$ 113, pHAV$_{LB}$ 1307, pHAV$_{LB}$ 228, pHAV$_{LB}$ 148, and pHAV$_{LB}$ 207 overlap to generate a map of about 7.4 kb, which represents at least 99% of the genome of HAV. Inserts from other clones (as indicated in Fig. 6) provided confirmation of overlap regions except for that between pHAV$_{LB}$ 228 and pHAV$_{LB}$ 148. Nick translated pHAV$_{LB}$ 228 insert hybridized to the 1.5 kb Pst I fragment of pHAV$_{LB}$ 148. The overlap as drawn is based on the assumption that there is only one Hinc II site, present in both clones, in the area about 4 kb from the 5' terminus of the genome. No other restriction sites in this region were found by digestion with over 30 other enzymes which have 5 and 6 base recognition sites. Heterogeneity in one restriction site (Nde I) was detected in the region about 2.5 kb from the 5' end of the viral RNA.

Extent of RNA sequence not represented in cDNA clones was determined by primer extension of HAV-infected AGMK cytoplasmic RNA. Racaniello, V. R. and Baltimore, D., Proc. Natl. Acad. Sci. USA 78 :4887-4891(1981).

A labelled Pst I/Bam HI fragment (0.2 to 0.7 kb in Fig. 6) from pHAV$_{LB}$ 113 was used as a primer for cDNA synthesis. When the template was cytoplasmic RNA from HAV-infected AGMK cells, the primer was extended to give a discrete band. There was no primer extension when RNA from uninfected AGMK cells was used as a template. When a labeled fragment from closer to the 5' end of the genome was used (Nco I at 0.05kb in Fig. 6 to Nci I at 0.25kb) as primer for viral RNA template that was purified from HAV-infected marmoset liver, the primer was similarly extended to a discrete length. Assuming that the 5' termini from HAV RNAs of different passage levels are conserved, we calculated that cloned cDNA extended to within 30 bases of the 5' end of the genome.

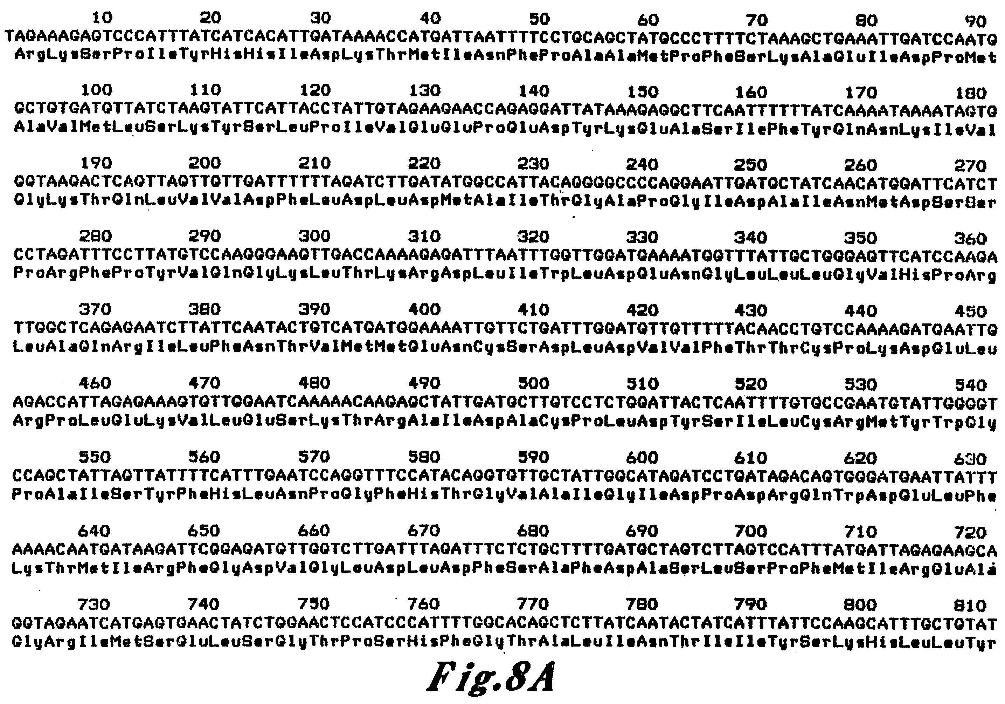

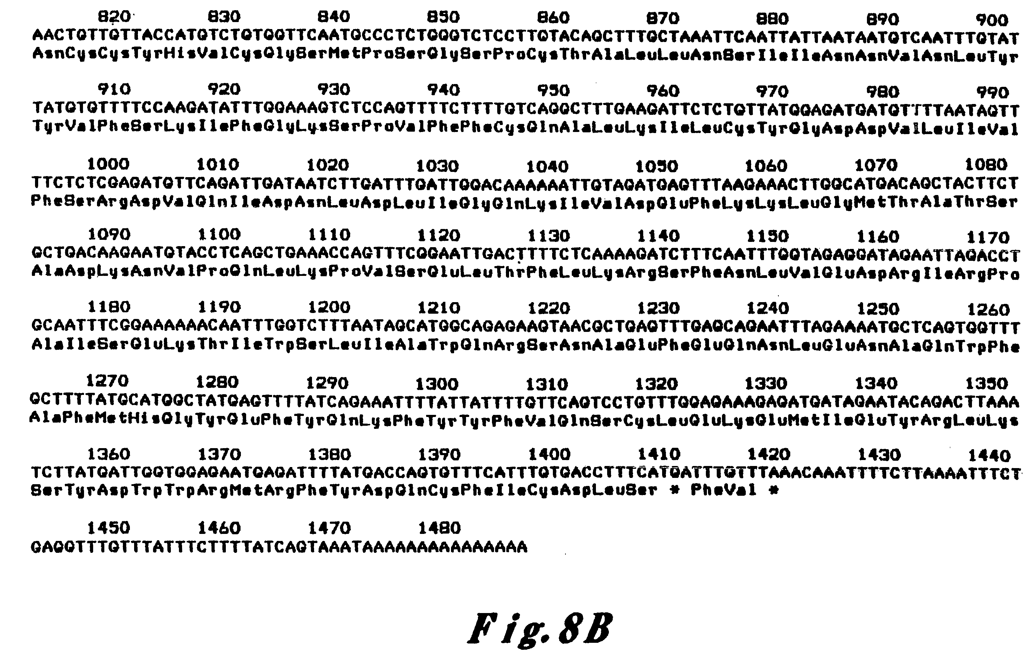

Partial Sequence of HAV cDNA. Sequence of cloned HAV cDNA was determined by the Method of Maxam and Gilbert. See, Maxam, A. M. & Gilbert, W., Methods Enzymol. 65 :499-560 (1980). The sequences presented in Figs. 7 and 8 were determined from individual cDNAs. Because the HAV stock used was not cloned virus, variations may exist between cDNAs that reflect variants in the stock.

In Fig. 7 nucleotide sequence is presented from pHAV$_{LB}$ 113, pHAV$_{LB}$ 39, pHAV$_L$ 1307, and pHAV$_{LB}$ 58 in the region corresponding to the 5' portion of HAV RNA. Base 1 corresponds to approximately 0.03kb on the scale in Fig. 6 and base 23 is the cleavage site for Nco I at 0.05kb. Codons for translation intiation (ATG) are found at bases 713-715 and 719-721 which are followed by open reading frame for the length of the sequence. The beginning of the long open reading frame, about 750 bases from the 5' end of the genome, corresponds to that for poliovirus RNA. See, Racaniello and Baltimore (1981). The sequences surrounding the putative initiation codons correspond to those described as consensus translation initiation sites in eukaryotic sequences by Kozak (Microbiological Reviews 47:1, 1983). Base 2792 is the Nco I site at 2.8 kb in Fig. 6. The predicted HAV amino acid sequences for this region have been compared, using computer programs, with the known capsid protein sequences of other piconaviruses (poliovirus, foot and mouth disease virus, and encephalomyocarditis virus) and locally homologous areas have been discovered. These results support the concept that HAV has a genome organization similar to that of the other piconaviruses, with capsid proteins derived from the amino-terminal portion of the genome polyprotein (sequences shown in Fig. 7).

The sequence of cloned cDNA from pHAV$_{LB}$ 93 corresponding to 6.0-7.5 kb in Fig. 6 and the 3' end of HAV RNA is presented in Fig. 8. The Pst I cleavage site (base 55) is at 6.0 kb in Fig. 6. There is an open reading frame that extends from the beginning of the sequence to a pair of nonsense codons that are separated by six bases (bases 1409-1420). The second nonsense codon is followed by 51 bases that do not contain an open reading frame initiated by ATG, followed by poly(A), presumed to represent the 3' end of the genome. Using computer programs for comparing amino acid sequences as described above, areas homologous to local regions of the poliovirus RNA polymerase (and the corresponding regions of other piconavirus genomes) have been discovered. In all of the sequences presented above, numerous stop codons in the other reading frames preclude a significant translation product.

EXAMPLE 2

DETECTION OF HAV RNA WITH LABELED HAV cDNA

Source of RNA

RNA was extracted from African green monkey kidney (AGMK) monolayers, either uninfected or infected with HAV as described in Example 1. RNA was also extracted from fecal suspensions from marmosets that had been experimentally infected with the HM175 strain of HAV. RNA was also extracted from serum or fecal suspensions from humans infected with a variety of HAV strains.

RNA Extraction

All solutions were treated with 0.1% diethyl-pyrocarbonate prior to use. Total cytoplasmic RNA was isolated from uninfected and HAV-infected AGMK cells. See Maniatis, T. et al. Molecular Cloning, Cold Spring Harbor Laboratory, Cold Spring Harbor, NY, 1982. Cells were recovered from monolayers treated with trypsin (20 ug/ml) in 20 mM Tris-HCl (pH 7.4), 0.15 M NaCl, and 1 mM EDTA by centrifugation at 2000 g for 5 min at 4°C. Cell pellets were resuspended in lysis buffer (10 mM Tris-HCl [pH 8.6], 0.14 M NaCl, 1.5 mM MgCl$_2$, 0.5% NP-40) containing RNasin (1000 units/ml), vortexed, layered over lysis buffer containing 24% sucrose and 1% NP-40, and centrifuged at 10,000 g for 20 min at 4° C. The resulting upper layer was saved, added to an equal volume of 0.2 M Tris-HCl (pH 7.5), 25 mM EDTA, 0.3 M NaCl, 2% NaDodSO₄, proteinase K (200 ug/ml) , and incubated at 37° C for 30 min. The mixture was extracted with phenol and with chloroform/isoamyl alcohol (24:1, vol:vol). RNA was precipitated from the aqueous phase with ethanol and analyzed by ultraviolet spectroscopy. RNA was similarly extracted from serum and fecal specimens, except that the initial cell lysis and centrifugation steps were deleted. RNA samples were adjusted to 250 ug/ml in 1 mM Tris-HCl (pH 7.5), 1 mM NaCl, 0.02 mM EDTA. Some such samples were digested with ribonuclease A (20 ug/ml) for 20 min at room temperature.

## Immobilization of RNA on Nitrocellulose Paper

Samples were applied to nitrocellulose paper using the "dot blot" technique. See, Thomas, P.S., Proc. Natl. Acad. Sci USA 77 :5201-5205(1980). Nitrocellulose paper was wetted in deionized water, saturated with 3 M NaCl, 0.3 M trisodium citrate; dried under a lamp; and ruled into 1.5 cm squares with a pencil. A series of 10-fold dilutions of RNA preparations (described above) was made. From each, 4 ul was directly applied into the center of a square on the nitrocellulose sheet resulting in rows of RNA "dots" containing from 1 pg to 1 ug of RNA. Alternatively, the aqueous phase from samples to be applied to a "slot blot" apparatus (see Wahl, Sequences Application Update #371, Schleider & Schuell Inc., Keene, N.H. 1983) were added to three volumes of 6.15M formaldehyde, 1.5M NaCl, 0.15M trisodium citrate. Samples were heated at 65° for 15 min., then applied using suction onto nitrocellulose paper in a filter manifold (Schleider & Schuell Minifold^R II). Buffer (l.5M NaC1, 0.15M trisodium citrate, pH 7.0) was used to presoak the nitrocellulose and wash the sample wells. After drying, the nitrocellulose sheet was baked under vacuum at 80° C for 2 hr and, prior to hybridization, wetted in 0.75 M NaCl, 0.075 M trisodium citrate, 50 mM sodium phosphate (pH 6.5) and incubated for 12 hr at 42° C in 50% vol/vol formamide, 0.75 M NaCl, 0.075 M trisodium citrate, 50 mM sodium phosphate (pH 6.5), 0.2% NaDodSO₄, denatured sheared salmon sperm DNA (100 ug/ml), 0.1% each: bovine serum albumin/polyvinylpyrollindone/Ficoll.

## Preparation of DNA Probe

Plasmid pHAV$_{LB}$ 228 was digested with Pst I. After electrophoresis in low melting point agarose, the cloned HAV cDNA insert was isolated and, after a second electrophoretic separation, isolated again from low melting point agarose. It was labeled by nick translation with ³²P to 2 to 6 x 10⁸ dpm/ug. Other probes from different regions of the HAV genome have been used with similar results.

## Hybridization and Autoradiography

Denatured DNA probe (10 ng/ml) was sealed in a plastic bag containing the nitrocellulose sheet with immobilized RNA and 50% vol/vol formamide, 0.75 M NaCl, 0.075 M trisodium citrate, 50 mM sodium phosphate (pH 6.5), 0.2% NaDodSO₄, denatured sheared salmon sperm DNA (100 ug/ml), 0.04% each: bovine serum albumin/polyvinylpyrollidone Ficoll for 36 hr at 42° C.

Unbound DNA probe was removed from the nitro-cellulose paper by successive washes in 15 mM NaCl, 1.5 mM trisodium citrate, 0.1% NaDodSO₄ for 15 min at 50° C until samples of wash buffer assayed in a liquid scintillation counter indicated that no further removal of probe was taking place. X-ray film was exposed to the nitrocellulose paper with an intensifying screen at -70° C for varying periods. When RNA from tissue culture was assayed, maximum sensitivity and specificity were obtained using a 17 hr exposure, when only samples from HAV-infected cells indicated binding of probe. No probe bound to those RNA samples that were treated with ribonuclease. In one series of dilutions of RNA from HAV-infected cells, probe binding was detectable with as little as 1 ng total cytoplasmic RNA. Since HAV RNA probably represents less than 1% of the total cytoplasmic RNA in an infected cell, approximately 10⁶ molecules were detected in this experiment. Results from marmoset and human samples of feces and serum showed that HAV RNA was detected with greater sensitivity than the detection of HAV by immune electron microscopy or by radiommunoassay. HAV RNA was detected to the level of approximately 1,000 chimpanzee infectious units from a variety of HAV strains.

Cell Deposits

The following cell strains containing plasmids designated pHAV, have been deposited at The American Type Culture Collection, Rockville, MD:

| Strain | pHAV | ATCC Number |
|--------|-------|-------------|
| LB58 | LB58 | 39454 |
| LB113 | LB113 | 39455 |
| LB148 | LB148 | 39456 |
| LB207 | LB207 | 39457 |
| LB228 | LB228 | 39458 |
| L1307 | L1307 | 39459. |

Industrial Applicability

The invention described herein is useful in the production of HAV cDNA by recombinant DNA techniques. HAV cDNA is in turn useful in assays for the detection of HAV, in the production of viral antigens, and in the production of antibodies against such antigens.

## Claims

1. A clonal cell line transformed with a recombinant DNA vector containing $HAV_{LB}$ 113 consisting essentially of those nucleotides of Figures 7A and 7B which correspond to the respective lengths shown in the restriction maps of Figure 6.

2. A clonal cell line transformed with a recombinant DNA vector containing $HAV_L$ 1307 consisting essentially of those nucleotides of Figures 7A, 7B, 7C, 7D and 7E which correspond to the respective lengths shown in the restriction maps of Figure 6.

3. A clonal cell line transformed with a recombinant DNA vector containing any one of :

   (a) $HAV_{LB}$ 58 (ATCC 39454)

   (b) $HAV_{LB}$ 113 (ATCC 39455)

   (c) $HAV_{LB}$ 148 (ATCC 39456)

   (d) $HAV_{LB}$ 207 (ATCC 39457)

   (e) $HAV_{LB}$ 228 (ATCC 39458)

   (f) $HAV_L$ 1307 (ATCC 39459)

4. A cell line according to any one of claims 1, 2 and 3, wherein the vector is selected from a bacterial plasmid, a phage, an animal virus or a yeast vector.

5. A cell line according to any one of claims 1, 2 and 3, wherein the vector is the chimeric bacterial plasmid pBR322.

6. A cell line according to any one of claims 1, 2 and 3, wherein the cell line is bacterial.

7. A cell line according to claim 6, wherein the bacterial cells comprise transformed E. coli cells.

8. HAV cDNA derived from a cell line according to any one of claims 1 to 7.

9. An assay for HAV characterised in that cDNA according to claim 8 is labeled and employed to bind to any HAV RNA present in a sample; and any labeled cDNA bound to said HAV RNA is detected.

10. An assay for the detection of HAV in a sample (e.g. a sample selected from human physiological samples, laboratory animal physiology samples, environmental samples and tissue culture samples), comprising:
   a) isolating an RNA-containing fraction of said sample, said RNA-containing fraction being a fraction which would contain HAV RNA if HAV were originally present in said sample;
   b) labeling HAV cDNA according to claim 8;
   c) incubating said labeled cDNA with said RNA-containing fraction in an incubation mixture under conditions sufficient for labeled cDNA to hybridize to HAV RNA;
   d) removing unbound labeled cDNA from the incubation mixture; and
   e) detecting remaining bound labeled cDNA.

11. An HAV antigen produced from HAV cDNA according to claim 8.

12. An antibody against HAV produced from HAV cDNA according to claim 8.

CLAIMS FOR THE CONTRACTING STATE AUSTRIA:

1. A process for preparing a clonal cell line, which process comprises transforming a cell line with a recombinant DNA vector containing $HAV_{LB}$ 113 consisting essentially of those nucleotides of Figures 7A and 7B which correspond to the respective lengths shown in the restriction maps of Figure 6.

2. A process for preparing a clonal cell line, which process comprises transforming a cell line with a recombinant DNA vector containing $HAV_L$ 1307 consisting essentially of those nucleotides of Figures 7A, 7B, 7C, 7D and 7E which correspond to the respective lengths shown in the restriction maps of Figure 6.

3. A process for preparing a clonal cell line, which process comprises transforming a cell line with a recombinant DNA vector containing any one of :

$$(a) \quad HAV_{LB} \ 58 \ (ATCC \ 39454)$$

$$(b) \quad HAV_{LB} \ 113 \ (ATCC \ 39455)$$

$$(c) \quad HAV_{LB} \ 148 \ (ATCC \ 39456)$$

$$(d) \quad HAV_{LB} \ 207 \ (ATCC \ 39457)$$

$$(e) \quad HAV_{LB} \ 228 \ (ATCC \ 39458)$$

$$(f) \quad HAV_L \ 1307 \ (ATCC \ 39459)$$

4. A process according to any one of claims 1, 2 and 3, wherein the vector is selected from a bacterial plasmid, a phage, an animal virus or a yeast vector.

**5.** A process according to any one of claims 1, 2 and 3, wherein the vector is the chimeric bacterial plasmid pBR322

**6.** A process according to any one of claims 1, 2 and 3, wherein the cell line is bacterial.

**7.** A process according to claim 6, wherein the bacterial cells comprise transformed E.coli cells.

**8.** A process for preparing HAV cDNA, which process comprises isolating said HAV cDNA from a clonal cell line produced according to any one of claims 1 to 7.

**9.** An assay for HAV characterised in that cDNA according to claim 8 is labeled and employed to bind to any HAV RNA present in a sample; and any labeled cDNA bound to said HAV RNA is detected.

**10.** An assay for the detection of HAV in a sample (e.g. a sample selected from human physiological samples, laboratory animal physiology samples, environmental samples and tissue culture samples), comprising :
a) isolating an RNA-containing fraction of said sample, said RNA-containing fraction being a fraction which would contain HAV RNA if HAV were originally present in said sample;
b) labeling HAV cDNA according to claim 8;
c) incubating said labeled cDNA with said RNA-containing fraction in an incubation mixture under conditions sufficient for labeled cDNA to hybridize to HAV RNA;
d) removing unbound labeled cDNA from the incubation mixture; and
e) detecting remaining bound labeled cDNA.

**11.** A process for preparing an HAV antigen, which process comprises producing said antigen from HAV cDNA wherein said HAV cDNA is prepared according to claim 8.

**12.** A process for preparing an antibody against HAV which process comprises producing said antibody from HAV cDNA wherein said HAV cDNA is prepared according to claim 8.

## Revendications

**1.** Lignée cellulaire clonale transformée à l'aide d'un vecteur ADN recombinant, caractérisée en ce qu'elle contient de l'HAV$_{LB}$ 113, et qu'elle consiste essentiellement en les nuoléotides des figures 7A et 7B, qui correspondent aux longueurs respectives représentées aux cartes de restriction de la figure 6.

**2.** Lignée cellulaire clonale tronsformée à l'aide d'un vecteur ADN recombinant, caractrisée en ce qu'elle contient do l'HAVL 1307, et qu'elle consiste essentiellement en les nucléotide des figures 7A, 7B, 7C. 7D et 7E, qui correspondent aux longueurs respectives représentées aux cartes de restriction de la figure 6.

**3.** Lignée cellulaire clonale transformée à l'aide d'un vecteur ADN recombinant, caractérisée par le fait qu'elle contient l'un quelconque de :

(a) HAV$_{LB}$ 58    (ATCC 39454)

(b) HAV$_{LB}$ 113    (ATCC 39455)

(c) HAV$_{LB}$ 148    (ATCC 39456)

(d) HAV$_{LB}$ 207    (ATCC 39457)

(e) HAV$_{LB}$ 228    (ATCC 39458)

(f) HAV$_L$ 1307    (ATCC 39459)

**4.** Lignée cellulaire selon l'une quelconque des revendications 1, 2 et 3 caractérisée par le fait que le vecteur est choisi parmi un plasmide bactérien, un phage, un virus animal ou un vecteur levure.

**5.** Lignée cellulaire selon l'une quelconque des revendications 1, 2 et 3 caractérisée par le fait que le vecteur est le plasmide bactérien chimérique pBR322.

**6.** Lignée cellulaire selon l'une quelconque des revendications 1, 2 et 3 caractérisé par le fait qui la lignée cellulaire est bactérienne.

**7.** Lignée celluloire selon la revendication 6, caractérisée par le fait que les cellules bactériennes comprennent dos cellules transformées E. Coli.

**8.** $ADN_C$ HAV caractérisé par le fait qu'il dérive d'une lignée cellulaire selon l'une quelconque des revendications 1 à 7.

**9.** Test pour l'HAV caractérisé on ce que l'$ADN_C$ selon la revendication 8 est marqué et utilisé pour se lier à tout ADN HAV présent dans un échantillon, toute liaison de l'$ADN_C$ marqué audit ARN HAV étant détectée.

**10.** Test pour la détection d'HAV dans un échantillon (tel qu'un échantillon prélevé sur des échantillons physiologiques humains, des échantillons physiologique animaux de laboratoire, des échantillons de l'environnement et des échantillons de culture tissulaire) caractérisé par le fait qu'Il comprend les étapes suivantes consistant à :
  a) isoler une fraction contenant de l'ARN dudit échantillon, ladite fraction contenant de l'ARN étant une fraction qui devrait contenir de l'ARN HAV si de l'HAV était présent à l'origine dans ledit échantillon;
  b) marquer l'$ADN_C$ HAV selon la revendication 8;
  c) faire incuber ledit $ADN_C$ marqué avec ladite fraction contenant de l'ARN dans un mélange d'incubation dans des conditions suffisantes pour que l'$ADN_C$ marqué hybride l'ARN HAV;
  d) extraire l'$ADN_C$ marqué non lié du mélange d'incubation; et
  e) détecter l'$ADN_C$ marqué resté lié.

**11.** Antigene HAV caractérisé par le fait qu'il est produit à partir d'$ADN_C$ HAV selon la revendication 8.

**12.** Anticorps contre l'HAV caractérisé par le fait qu'il est produit à partir d'$ADN_C$ HAV selon la revendication 8.

Revendications pout l'Etat contractant AT:

**1.** Procédé de préparation d'une lignée cellulaire clonale, ledit procédé étant caractérisé par le fait qu'il comprend la transformation d'une lignée cellulaire avec un vecteur ADN recombinant contenant de l'$HAV_{LB}$ 113, la lignée consistant essentiellement en les nucléotides des figures 7A et 7B qui correspondent aux longueurs respectives représentées aux cartes de restriction de la figure 6.

**2.** Procédé de préparation d'une lignée cellulaire clonale, ledit procédé étant caractérisé par le fait qu'il comprend la transformation d'une lignée cellulaire avec un vecteur ADN recombinant contenant de l'$HAV_C$ 1303 consistant essentiellement en les nucléotides des figures 75, 7B, 7C, 7D et 7E, qui correspondant aux longueurs respectives représentées aux cartes de restriction de la figure 6.

**3.** Procédé de préparation d'une lignée cellulaire clonale, ledit procédé étant caractérisé par le fait qu'il comprend la' transformation d'une lignée cellulaire avec un vecteur ADN recombinant contenant l'un quelconque de :

(a) HAV$_{LB}$ 58    (ATCC 39454)

(b) HAV$_{LB}$ 113   (ATCC 39455)

(c) HAV$_{LB}$ 148   (ATCC 39456)

(d) HAV$_{LB}$ 207   (ATCC 39457)

(e) HAV$_{LB}$ 228   (ATCC 39458)

(f) HAV$_L$ 1307 (ATCC 39459)

4. Procédé selon l'une quelconque des revendications 1, 2 et 3 caractérisé par le fait que le vecteur est choisi parmi un plasmide bactérien, un phage, un virus animal ou un vecteur levure.

5. Procédé selon l'une quelconque des revendications 1, 2 et 3 caractérisé par le fair que le vecteur est le plasmide bactérien chimérique pBR322.

6. Procédé selon l'une quelconque des revendications 1, 2 et 3 caractérisé par le fait que la lignée cellulaire est bactérienne.

7. Procédé selon la revendication 6 caracterisé par le fait que la lignée cellulaire est bactérienne.

8. Procédé de préparation ou d'ADN$_C$ HAV caractérisé par le fait qu'il comprend l'isolement dudit ADN$_C$ HAV d'une lignée cellulaire clonale produite selon l'une quelconque des revendications 1 à 7.

9. Test pour l'HAV caractérisé par le fait que l'ADN$_C$ selon la revendication 8 est marqué d'une lignée cellulaire selon l'une quelconque des revendications 1 à 7.

10. Test pour la détection d'HAV dans un échantillon (tel qu'un échantillon prélevé sur des échantillons physiologiques humains, des échantillons physionomiques animaux de laboratoire, des échantillons de l'environnement et des échantillons de culture tissulaire), caractérisé par le fait qu'il comprend les étapes suivantes consistant à:
   a) isoler une fraction contenant de l'ARN dudit échantillon, ladite fraction contenant de l'ARN étant une fraction qui devrait contenir de l'ARN HAV si de l'HAV était présent à l'origine dans ledit échantillon;
   b) marquer l'ADN$_C$ HAV selon la revendication 8;
   c) faire incuber ledit ADN$_C$ marqué avec ladite fraction contenant de l'ARN dans un mélange d'incubation dans des conditions suffisantes pour que l'ADN$_C$ marqué hybride l'ARN HAV;
   d) extraire l'ADN$_C$ marqué non lié du mélange d'incubation; et
   e) détecter l'ADN$_C$ marqué resté lié.

11. Procédé de préparation d'un antigène HAV caractérisé par le fait qu'il comprend la préparation dudit antigène à partir d'ADNXC HAV, selon la revendication 8.

12. Procédé de préparation d'un anticorps contre l'HAV caractérisé par le fait qu'il comprend la préparation dudit anticorps à partir d'ADN$_C$ HAV selon la revendication 8.

## Ansprüche

1. Klonale Zellinie, ungewandelt mit einen rekombinanten DNA-Vektor, der HAV$_{LB}$ 113 enthält, im wesentlichen bestehend aus denjenigen Nukleotiden der Fig. 7A und 7B, die den in den Restriktionskarten der Fig. 6 gezeigten jeweiligen Längen entsprechen.

2. Klonale Zellinie, umgewandelt mit einem rekombinanten DNA-Vektor, der HAV$_L$307 enthält, im wesentli-

EP 0 162 054 B1

chen bestehend aus denjenigen Nukleotiden der Figuren 7A, 7B, 7C, 7D und 7E, die den in den Restriktionskarten der Figur 6 gezeigten jeweiligen Längen entsprechen.

3. Klonale Zellinie, umgewandelt mit einem rekombinanten DNA-Vektor, der einen beliebigen von

$$
\begin{array}{ll}
\text{(a)} & \text{HAV}_{LB} \ 58 \ (\text{ATCC } 39454) \\
\text{(b)} & \text{HAV}_{LB} \ 113 \ (\text{ATCC } 39455) \\
\text{(c)} & \text{HAV}_{LB} \ 148 \ (\text{ATCC } 39456) \\
\text{(d)} & \text{HAV}_{LB} \ 207 \ (\text{ATCC } 39457) \\
\text{(e)} & \text{HAV}_{LB} \ 228 \ (\text{ATCC } 39458) \\
\text{(f)} & \text{HAV}_{L} \ 1307 \ (\text{ATCC } 39459)
\end{array}
$$

enthält.

4. Zellinie nach einem der Ansprüche 1, 2 und 3, bei der der Vektor aus einem bakteriellen Plasmid, einem Phage, einem tierischen Virus oder einem Hefevektor ausgewählt ist.

5. Zellinie nach einen der Ansprüche 1, 2 und 3, bei der der Vektor das chimärische Bakterienplasmid pBR322 ist.

6. Zellinie nach einem der Ansprüche 1, 2 und 3, bei der die Zellinie bakteriell ist.

7. Zellinie nach Anspruch 6, bei der die Bakterienzellen aus umgewandelten E.-coli-Zellen bestehen.

8. HAV-cDNA, abgeleitet aus einer Zellinie nach einem der Ansprüche 1 bis 7.

9. Untersuchung auf HAV, dadurch gekennzeichnet, daß cDNA gemäß Anspruch 8 markiert und dazu verwendet wird, in der Probe vorhandene HAV-RNA zu binden, und daß an die HAV-RNA gebundene markierte cDNA festgestellt wird.

10. Untersuchung zur Feststellung von HAV in einer Probe, (z.B. ausgewählt aus menschlichen physiologischen Proben, Tierversuchsphysiologieproben, Umgebungsproben und Gewebekulturproben), darin bestehend. daß
   a) eine RNA-haltige Fraktion der Probe isoliert wird, wobei die RNA-haltige Fraktion eine Fraktion ist. die HAV-RNA enthalten würde, falls HAV ursprünglich in der Probe vorhanden wäre.
   b) HAV-cDNA nach Anspruch 8 markiert wird,
   c) die markierte cDNA mit der RNA-haltigen Fraktion in einem Inkubationsgemisch unter solchen Bedingungen inkubiert wird. daß markierte cDNA zu HAV-RNA hybridisiert wird,
   d) ungebundene markierte cDNA aus dem Inkubationsgemisch entfernt wird und
   e) die verbleibende gebundene markierte cDNA festgestellt wird.

11. HAV-Antigen. hergestellt aus HAV-cDNA gemäß Anspruch 8.

12. Antikörper gegen HAV, hergestellt aus HAV-cDNA gemäß Anspruch 8.

Patentansprüche für den Vertragsstaat: AT

1. Verfahren zur Herstellung einer klonalen Zellinie, welches darin besteht, daß eine Zellinie mit einem rekombinanten DNA-Vektor umgewandelt wird, der HAV$_{LB}$ 113 enthält, im wesentlichen bestehend aus denjenigen Nukleotiden der Figuren 7A und 7B, die den in den Restriktionskarten der Figur 6 gezeigten jeweiligen Längen entsprechen.

2. Verfahren zur Herstellung einer klonalen Zellinie, welches darin besteht, daß eine Zellinie mit einem rekombinanten DNA-Vektor umgewandelt wird, der HAVL 1307 enthält, im wesentlichen bestehend aus

18

denjenigen Nukleotiden der Figuren 7A, 7B, 7C, 7D und 7E, die den in den Restriktionskarten der Figur 6 gezeigten jeweiligen Längen entsprechen.

3. Verfahren zur Herstellung einer klonalen Zellinie, welches darin besteht, daß eine Zellinie mit einem rekombinanten DNA-Vektor umgewandelt wird, der einen beliebigen von

$$(a) \quad HAV_{LB} \ 58 \ (ATCC39454)$$
$$(b) \quad HAVLB \ 113 \ (ATCC \ 39455)$$
$$(c) \quad HAV_{LB} \ 148 \ (ATCC \ 39456)$$
$$(d), \ HAV_{LB} \ 207 \ (ATCC \ 39457)$$
$$(e) \quad HAV_{LB} \ 228 \ (ATCC \ 39458)$$
$$(f) \quad HAV_{L} \ 1307 \ (ATCC \ 39459)$$

enthält.

4. Verfahren nach einem der Ansprüche 1, 2 und 3, bei dem der Vektor aus einem bakteriellen Plasmid, einem Phage, einem tierischen Virus oder einem Hefevektor ausgewählt wird.

5. Verfahren nach einem der Ansprüche 1, 2 und 3, bei dem der Vektor das chimärische Bakterienplasmid pBR322 ist.

6. Verfahren nach einem der Ansprüche 1, 2 und 3, bei dem die Zellinie bakteriell ist.

7. Verfahren nach Anspruch 6, bei der die Bakterienzellen aus umgewandelten E.-coli-Zellen bestehen.

8. Verfahren zur Herstellung von HAV-cDNA, welches darin besteht, daß die HAV-cDNA aus einer nach einem der Ansprüche 1 bis 7 hergestellten klonalen Zellinien isoliert wird.

9. Untersuchung auf HAV, dadurch gekennzeichnet, daß cDNA gemäß Anspruch 8 markiert und dazu verwendet wird, in der Probe vorhandene HAV-RNA zu binden, und daß an die HAV-RNA gebundene markierte cDNA festgestellt wird.

10. Untersuchung zur Feststellung von HAV in einer Probe, (z.B. ausgewählt aus menschlichen physiologischen Proben, Tierversuchsphysiologieproben, Umgebungsproben und Gewebekulturproben), darin bestehend, daß
   a) eine RNA-haltige Fraktion der Probe isoliert wird, wobei die RNA-haltige Fraktion eine Fraktion ist, die HAV-RNA enthalten würde, falls HAV ursprünglich in der Probe vorhanden wäre,
   b) HAV-cDNA nach Anspruch 8 markiert wird,
   c) die markierte cDNA mit der RNA-haltigen Fraktion in einem Inkubationsgemisch unter solchen Bedingungen inkubiert wird, daß markierte cDNA zu HAV-RNA hybridisiert wird,
   d) ungebundene markierte cDNA aus dem Inkubationsgemisch entfernt wird und
   e) die verbleibende gebundene markierte cDNA festgestellt wird.

11. Verfahren zur Herstellung eines HAV-Antigens, welches darin besteht, daß das Antigen aus HAV-cDNA hergestellt wird, wobei die HAV-cDNA gemäß Anspruch 8 hergestellt wird.

12. Verfahren zur Herstellung eines Antikörpers gegen HAV, welches darin besteht, daß der Antikörper aus HAV-cDNA hergestellt wird, wobei die HAV-cDNA gemäß Anspruch 8 hergestellt wird.

Fig.1

OBTAIN A SAMPLE TO BE
ASSAYED FOR THE PRESENCE
OF HEPATITIS A

ISOLATE AN RNA FRACTION FROM
THE SAMPLE WHICH WOULD CONTAIN
HEPATITIS A, IF PRESENT

LABEL HEPATITIS A cDNA

INCUBATE LABELED HEPATITIS A cDNA
WITH THE RNA FRACTION UNDER CONDITIONS
SUFFICIENT FOR THE cDNA TO BIND
TO HEPATITIS A, IF PRESENT

REMOVE UNBOUND HEPATITIS A cDNA
FROM LABELED BOUND HEPATITIS A

DETECT LABELED HEPATITIS A cDNA
BOUND TO HEPATITIS A RNA

*Fig.2*

*Fig.3*

*Fig. 4*

*Fig.5*

Fig. 6

```
         10        20        30        40        50        60        70        80        90
TTCCGGAGTCCCTCTTGGAAGTCCATGGTGAGGGGACTTGATACCTCACCGCCGTTTGCCTAGGCTATAGGCTAAATTTTCCCTTTCCCTT

        100       110       120       130       140       150       160       170       180
TTCCCTTTCCTATTCCCTTTGTTTTGCTTGTAAATATTAATTCCTGCAGGTTCAGGGTTCTTAAATCTGTTTCTCTATAAGAACACTCAT

        190       200       210       220       230       240       250       260       270
TTTTCACGCTTTCTGTCTTCTTTCTTCCAGGGCTCTCCCCTTGCCCTAGGCTCTGGCCGTTGCGCCCGGCGGGGTCAACTCCATGATTAG

        280       290       300       310       320       330       340       350       360
CATGGAGCTGTAGGAGTCTAAATTGGGGACACAGATGTTTGGAACGTCACCTTGCAGTGTTAACTTGGCTTTCATGAATCTCTTTGATCT

        370       380       390       400       410       420       430       440       450
TCCACAAGGGGTAGGCTACGGGTGAAACCTCTTAGGCTAATACTTCTATGAAGAGATGCCTTGGATAGGGTAACAGCGGCGGATATTGGT

        460       470       480       490       500       510       520       530       540
GAGTTGTTAAGACAAAAACCATTCAACGCCGGAGGACTGACTCTCATCCAGTGGATGCATTGAGTGGATTGACTGTCAGGGCTGTCTTTA

        550       560       570       580       590       600       610       620       630
GGCTTAATTCCAGACCTCTCTGTGCTTAGGGCAAACATCATTTGGCCTTAAATGGGATTCTGTGAGAGGGGATCCCTCCATTGACAGCTG

        640       650       660       670       680       690       700       710       720
GACTGTTCTTTGGGGCCTTATGTGGTGTTTGCCTCTGAGGTACTCAGGGGCATTTAGGTTTTTCCTCATTCTTAAATAATAATGAACATG
                                                                                     MetAsnMet
```

*Fig.7A*

EP 0 162 054 B1

```
        730       740       750       760       770       780       790       800       810
TCTAGACAAGGTATTTTCCAGACTGTTGGGAGTGGTCTTGACCACATCCTGTCTTTGGCAGACATTGAGGAAGAGCAAATGATTCAATCA
SerArgGlnGlyIlePheGlnThrValGlySerGlyLeuAspHisIleLeuSerLeuAlaAspIleGluGluGluGlnMetIleGlnSer

        820       830       840       850       860       870       880       890       900
GTTGATAGGACTGCAGTGACTGGTGCTTCTTATTTTACTTCTGTGGATCAATCTTCAGTTCATACAGCTGAGGTTGGATCACACCAGGTT
ValAspArgThrAlaValThrGlyAlaSerTyrPheThrSerValAspGlnSerSerValHisThrAlaGluValGlySerHisGlnVal

        910       920       930       940       950       960       970       980       990
GAACCTTTGAGAACCTCTGTTGATAAACCCGGTTCAAAGAAGACTCAGGGAGAGAAATTTTTCTTGATTCATTCTGCAGATTGGCTTACT
GluProLeuArgThrSerValAspLysProGlySerLysLysThrGlnGlyGluLysPhePheLeuIleHisSerAlaAspTrpLeuThr

        1000      1010      1020      1030      1040      1050      1060      1070      1080
ACACATGCTCTTTTCCATGAAGTTGCAAAATTGGATGTGGTGAAATTATTATACAATGAGCAGTTTGCTGTTCAAGGGTTGTTGAGATAC
ThrHisAlaLeuPheHisGluValAlaLysLeuAspValValLysLeuLeuTyrAsnGluGlnPheAlaValGlnGlyLeuLeuArgTyr

        1090      1100      1110      1120      1130      1140      1150      1160      1170
CATACATATGCAAGATTTGGCATTGAAATTCAAGTTCAGATAAACCCTACACCTTTCCAACAGGGGGGATTGATCTGTGCTATGGTTCCT
HisThrTyrAlaArgPheGlyIleGluIleGlnValGlnIleAsnProThrProPheGlnGlnGlyGlyLeuIleCysAlaMetValPro

        1180      1190      1200      1210      1220      1230      1240      1250      1260
GGTGACCAGAGCTATGGTTCTATAGCATCATTGACTGTTTATCCTCATGGTTTGTTAAATTGCAATATTAACAATGTGGTTAGAATAAAG
GlyAspGlnSerTyrGlySerIleAlaSerLeuThrValTyrProHisGlyLeuLeuAsnCysAsnIleAsnAsnValValArgIleLys

        1270      1280      1290      1300      1310      1320      1330      1340      1350
GTTCCATTTATTTACACAAGAGGTGCTTACCACTTTAAAGATCCACAATACCCAGTTTGGGAATTGACAATTAGAGTTTGGTCAGAATTA
ValProPheIleTyrThrArgGlyAlaTyrHisPheLysAspProGlnTyrProValTrpGluLeuThrIleArgValTrpSerGluLeu

        1360      1370      1380      1390      1400      1410      1420      1430      1440
AATATTGGGACAGGAACTTCAGCTTATACTTCACTCAATGTTTAGCTAGATTTACAGATTTGGAGTTGCATGGATTAACTCCTCTTTCT
AsnIleGlyThrGlyThrSerAlaTyrThrSerLeuAsnValLeuAlaArgPheThrAspLeuGluLeuHisGlyLeuThrProLeuSer
```

*Fig.7B*

```
ACACAAATGATGAGAAATGAATTTAGGGTCAGTACTACTGAGAATGTGGTGAATCTGTCAAATTATGAAGATGCAAGAGCAAAGATGTCT
ThrGlnMetMetArgAsnGluPheArgValSerThrThrGluAsnValValAsnLeuSerAsnTyrGluAspAlaArgAlaLysMetSer

TTTGCTTTGGATCAGGAAGATTGGAAATCTGATCCGTCCCAGGGTGGTGGGATCAAAATTACTCATTTTACTACTTGGACATCTATTCCA
PheAlaLeuAspGlnGluAspTrpLysSerAspProSerGlnGlyGlyGlyIleLysIleThrHisPheThrThrTrpThrSerIlePro

ACTTTGGCTGCTCAGTTTCCATTTAATGCTTCAGACTCAGTTGGTCAACAAATTAAAGTTATTCCAGTTGACCCATATTTTTTCCAAATG
ThrLeuAlaAlaGlnPheProPheAsnAlaSerAspSerValGlyGlnGlnIleLysValIleProValAspProTyrPhePheGlnMet

ACAAATACGAATCCTGACCAAAAATGTATAACTGCTTTGGCTTCTATTTGTCAGATGTTTGTTTTTGGAGAGGAGATCTTGTCTTTGAT
ThrAsnThrAsnProAspGlnLysCysIleThrAlaLeuAlaSerIleCysGlnMetPheCysPheTrpArgGlyAspLeuValPheAsp

TTTCAAGTTTTTCCCACCAAATATCATTCAGGTAGATTACTGTTTTGTTTTGTTCCTGGCAATGAGCTAATAGATGTTTCTGGAATCACA
PheGlnValPheProThrLysTyrHisSerGlyArgLeuLeuPheCysPheValProGlyAsnGluLeuIleAspValSerGlyIleThr

TTAAAGCAAGCAACTACTGCTCCTTGTGCAGTAATGGATATTACAGGAGTGCAGTCAACTTTGAGATTTCGTGTTCCCTGGATTTCTGAC
LeuLysGlnAlaThrThrAlaProCysAlaValMetAspIleThrGlyValGlnSerThrLeuArgPheArgValProTrpIleSerAsp

ACTCCTTACAGAGTGAACAGGTATACAAAGTCAGCACATCAGAAAGGTGAGTACACTGCCATTGGGAAGCTTATTGTGTATTGTTATAAC
ThrProTyrArgValAsnArgTyrThrLysSerAlaHisGlnLysGlyGluTyrThrAlaIleGlyLysLeuIleValTyrCysTyrAsn

AGATTGACCTCTCCTTCTAACGTTGCTTCCCATGTCAGAGTGAATGTTTATCTTTCAGCAATTAACTTGGAATGTTTTGCTCCTCTTTAT
ArgLeuThrSerProSerAsnValAlaSerHisValArgValAsnValTyrLeuSerAlaIleAsnLeuGluCysPheAlaProLeuTyr
```

*Fig. 7C*

EP 0 162 054 B1

EP 0 162 054 B1

```
         2170      2180      2190      2200      2210      2220      2230      2240      2250
CATGCTATGGATGTTACTACACAAGTTGGAGATGATTCTGGAGGTTTTTTCAACAACAGTTTCTACAGAACAGAATGTTCCAGATCCCCAA
HisAlaMetAspValThrThrGlnValGlyAspAspSerGlyGlyPheSerThrThrValSerThrGluGlnAsnValProAspProGln

         2260      2270      2280      2290      2300      2310      2320      2330      2340
GTTGGTATAACAACCATGAAAGATTTGAAAGGAAAAGCTAACAGAGGGAAAATGGATGTTTCAGGAGTACAAGCACCTGTGGGAGCTATC
ValGlyIleThrThrMetLysAspLeuLysGlyLysAlaAsnArgGlyLysMetAspValSerGlyValGlnAlaProValGlyAlaIle

         2350      2360      2370      2380      2390      2400      2410      2420      2430
ACAACAATTGAGGATCCAGTTTTAGCAAAGAAAGTACCTGAGACATTTCCTGAATTGAAACCTGGAGAATCCAGACATACATCAGATCAT
ThrThrIleGluAspProValLeuAlaLysLysValProGluThrPheProGluLeuLysProGlyGluSerArgHisThrSerAspHis

         2440      2450      2460      2470      2480      2490      2500      2510      2520
ATGTCCATCTACAAGTTTATGGGAAGGTCTCATTTCTTGTGCACTTTTACATTCAATTCAAATAATAAAGAGTACACATTTCCTATAACC
MetSerIleTyrLysPheMetGlyArgSerHisPheLeuCysThrPheThrPheAsnSerAsnAsnLysGluTyrThrPheProIleThr

         2530      2540      2550      2560      2570      2580      2590      2600      2610
TTGTCTTCAACCTCTAATCCTCCTCATGGTTTGCCATCAACACTGAGGTGGTTTTTCAACTTGTTTCAGTTGTATAGAGGGCCTTTAGAT
LeuSerSerThrSerAsnProProHisGlyLeuProSerThrLeuArgTrpPhePheAsnLeuPheGlnLeuTyrArgGlyProLeuAsp

         2620      2630      2640      2650      2660      2670      2680      2690      2700
CTGACAATTATTATTACAGGAGCAACTGATGTAGATGGCATGGCCTGGTTCACTCCAGTAGGTCTTGCCGTTGATACTCCTTGGGTAGAG
LeuThrIleIleIleThrGlyAlaThrAspValAspGlyMetAlaTrpPheThrProValGlyLeuAlaValAspThrProTrpValGlu

         2710      2720      2730      2740      2750      2760      2770      2780      2790
AAGGAGTCAGCTTTGTCTATTGACTACAAAACTGCTCTTGGAGCTGTCAGATTTAACACAAGGAGAACAGGGAACATTCAGATTAGATTA
LysGluSerAlaLeuSerIleAspTyrLysThrAlaLeuGlyAlaValArgPheAsnThrArgArgThrGlyAsnIleGlnIleArgLeu

         2800      2810      2820      2830      2840      2850      2860      2870      2880
CCATGGTATTCTTATTTATATGCTGTGTCTGGAGCACTGGATGGTTTGGGTGACAAGACAGATTCTACATTTGGATTGGTTTCTATTCAG
ProTrpTyrSerTyrLeuTyrAlaValSerGlyAlaLeuAspGlyLeuGlyAspLysThrAspSerThrPheGlyLeuValSerIleGln
```

# *Fig.7D*

```
    2890      2900      2910      2920      2930      2940      2950      2960      2970
ATTGCAAATTACAATCATTCTGATGAATACTTGTCTTTTAGTTGTTATTTGTCTGTCACAGAACAATCAGAGTTTTATTTTCCCAGAGCT
IleAlaAsnTyrAsnHisSerAspGluTyrLeuSerPheSerCysTyrLeuSerValThrGluGlnSerGluPheTyrPheProArgAla

    2980      2990      3000      3010      3020      3030      3040      3050      3060
CCATTGAACTCAAATGCCATGTTATCCACTGAATCAATGATGAGCAGAATTGCAGCTGGAGACTTGGAGTCATCAGTGGATGATCCTAGA
ProLeuAsnSerAsnAlaMetLeuSerThrGluSerMetMetSerArgIleAlaAlaGlyAspLeuGluSerSerValAspAspProArg

    3070      3080      3090      3100      3110      3120      3130      3140      3150
TCAGAGGAAGATAAAAGATTTGAGAGTCATATAGAATGCAGGAAGCCATATAAAGAACTGAGATTAGAAGTTGGGAAACAAAGACTCAAG
SerGluGluAspLysArgPheGluSerHisIleGluCysArgLysProTyrLysGluLeuArgLeuGluValGlyLysGlnArgLeuLys

    3160      3170      3180      3190      3200      3210      3220      3230      3240
TATGCTCAGGAAGAATTGTCAAATGAAGTACTTCCACCCCCTAGGAAAATGAAGGGACTGTTTTCACAAGCCAAAATTTCTCTTTTTTAT
TyrAlaGlnGluGluLeuSerAsnGluValLeuProProProArgLysMetLysGlyLeuPheSerGlnAlaLysIleSerLeuPheTyr

    3250      3260      3270
ACTGAGGAGCATGAAATAATGAAGTTTTCCTGG
ThrGluGluHisGluIleMetLysPheSerTrp
```

*Fig.7E*

```
          10        20        30        40        50        60        70        80        90
TAGAAAGAGTCCCATTTATCATCACATTGATAAAACCATGATTAATTTTCCTGCAGCTATGCCCTTTTCTAAAGCTGAAATTGATCCAATG
ArgLysSerProIleTyrHisHisIleAspLysThrMetIleAsnPheProAlaAlaMetProPheSerLysAlaGluIleAspProMet

         100       110       120       130       140       150       160       170       180
GCTGTGATGTTATCTAAGTATTCATTACCTATTGTAGAAGAACCAGAGGATTATAAAGAGGCTTCAATTTTTTATCAAAATAAAATAGTG
AlaValMetLeuSerLysTyrSerLeuProIleValGluGluProGluAspTyrLysGluAlaSerIlePheTyrGlnAsnLysIleVal

         190       200       210       220       230       240       250       260       270
GGTAAGACTCAGTTAGTTGTTGATTTTTTAGATCTTGATATGGCCATTACAGGGGGCCCCAGGAATTGATGCTATCAACATGGATTCATCT
GlyLysThrGlnLeuValValAspPheLeuAspLeuAspMetAlaIleThrGlyAlaProGlyIleAspAlaIleAsnMetAspSerSer

         280       290       300       310       320       330       340       350       360
CCTAGATTTCCTTATGTCCAAGGGAAGTTGACCAAAAGAGATTTAATTTGGTTGGATGAAAATGGTTTATTGCTGGGAGTTCATCCAAGA
ProArgPheProTyrValGlnGlyLysLeuThrLysArgAspLeuIleTrpLeuAspGluAsnGlyLeuLeuLeuGlyValHisProArg

         370       380       390       400       410       420       430       440       450
TTGGCTCAGAGAATCTTATTCAATACTGTCATGATGGAAAATTGTTCTGATTTGGATGTTGTTTTTACAACCTGTCCAAAAGATGAATTG
LeuAlaGlnArgIleLeuPheAsnThrValMetMetGluAsnCysSerAspLeuAspValValPheThrThrCysProLysAspGluLeu

         460       470       480       490       500       510       520       530       540
AGACCATTAGAGAAAGTGTTGGAATCAAAAACAAGAGCTATTGATGCTTGTCCTCTGGATTACTCAATTTTGTGCCGAATGTATTGGGGT
ArgProLeuGluLysValLeuGluSerLysThrArgAlaIleAspAlaCysProLeuAspTyrSerIleLeuCysArgMetTyrTrpGly

         550       560       570       580       590       600       610       620       630
CCAGCTATTAGTTATTTTCATTTGAATCCAGGTTTCCATACAGGTGTTGCTATTGGCATAGATCCTGATAGACAGTGGGATGAATTATTT
ProAlaIleSerTyrPheHisLeuAsnProGlyPheHisThrGlyValAlaIleGlyIleAspProAspArgGlnTrpAspGluLeuPhe

         640       650       660       670       680       690       700       710       720
AAAACAATGATAAGATTCGGAGATGTTGGTCTTGATTTAGATTTCTCTGCTTTTGATGCTAGTCTTAGTCCATTTATGATTAGAGAAGCA
LysThrMetIleArgPheGlyAspValGlyLeuAspLeuAspPheSerAlaPheAspAlaSerLeuSerProPheMetIleArgGluAla

         730       740       750       760       770       780       790       800       810
GGTAGAATCATGAGTGAACTATCTGGAACTCCATCCCATTTTGGCACAGCTCTTATCAATACTATCATTTATTCCAAGCATTTGCTGTAT
GlyArgIleMetSerGluLeuSerGlyThrProSerHisPheGlyThrAlaLeuIleAsnThrIleIleTyrSerLysHisLeuLeuTyr
```

## Fig.8A

EP 0 162 054 B1

```
     820       830        840       850        860       870        880       890        900
AACTGTTGTTACCATGTCTGTGGTTCAATGCCCTCTGGGTCTCCTTGTACAGCTTTGCTAAATTCAATTATTAATAATGTCAATTTGTAT
AsnCysCysTyrHisValCysGlySerMetProSerGlySerProCysThrAlaLeuLeuAsnSerIleIleAsnAsnValAsnLeuTyr

     910       920        930       940        950       960        970       980        990
TATGTGTTTTCCAAGATATTTGGAAAGTCTCCAGTTTTCTTTTGTCAGGCTTTGAAGATTCTCTGTTATGGAGATGATGTTTTAATAGTT
TyrValPheSerLysIlePheGlyLysSerProValPhePheCysGlnAlaLeuLysIleLeuCysTyrGlyAspAspValLeuIleVal

     1000      1010       1020      1030       1040      1050       1060      1070       1080
TTCTCTCGAGATGTTCAGATTGATAATCTTGATTTGATTGGACAAAAAATTGTAGATGAGTTTAAGAAACTTGGCATGACAGCTACTTCT
PheSerArgAspValGlnIleAspAsnLeuAspLeuIleGlyGlnLysIleValAspGluPheLysLysLeuGlyMetThrAlaThrSer

     1090      1100       1110      1120       1130      1140       1150      1160       1170
GCTGACAAGAATGTACCTCAGCTGAAACCAGTTTCGGAATTGACTTTTCTCAAAAGATCTTTCAATTTGGTAGAGGATAGAATTAGACCT
AlaAspLysAsnValProGlnLeuLysProValSerGluLeuThrPheLeuLysArgSerPheAsnLeuValGluAspArgIleArgPro

     1180      1190       1200      1210       1220      1230       1240      1250       1260
GCAATTTCGGAAAAAACAATTTGGTCTTTAATAGCATGGCAGAGAAGTAACGCTGAGTTTGAGCAGAATTTAGAAAATGCTCAGTGGTTT
AlaIleSerGluLysThrIleTrpSerLeuIleAlaTrpGlnArgSerAsnAlaGluPheGluGlnAsnLeuGluAsnAlaGlnTrpPhe

     1270      1280       1290      1300       1310      1320       1330      1340       1350
GCTTTTATGCATGGCTATGAGTTTTATCAGAAATTTTATTATTTTGTTCAGTCCTGTTTGGAGAAAGAGATGATAGAATACAGACTTAAA
AlaPheMetHisGlyTyrGluPheTyrGlnLysPheTyrTyrPheValGlnSerCysLeuGluLysGluMetIleGluTyrArgLeuLys

     1360      1370       1380      1390       1400      1410       1420      1430       1440
TCTTATGATTGGTGGAGAATGAGATTTTATGACCAGTGTTTCATTTGTGACCTTTCATGATTTGTTTAAACAAATTTTCTTAAAATTTCT
SerTyrAspTrpTrpArgMetArgPheTyrAspGlnCysPheIleCysAspLeuSer * PheVal *

     1450      1460       1470      1480
GAGGTTTGTTTATTTCTTTTATCAGTAAATAAAAAAAAAAAAAAA
```

EP 0 162 054 B1

# Fig.8B